Europäisches Patentamt

European Patent Office

Office européen des brevets

(19)

(11) **EP 0 698 102 B1**

(12) **EUROPÄISCHE PATENTSCHRIFT**

(45) Veröffentlichungstag und Bekanntmachung des Hinweises auf die Patenterteilung:
**01.03.2006 Patentblatt 2006/09**

(51) Int Cl.:
*C12N 15/53* (2006.01)     *C12N 9/04* (2006.01)
*C12Q 1/60* (2006.01)

(21) Anmeldenummer: **94915569.1**

(22) Anmeldetag: **02.05.1994**

(86) Internationale Anmeldenummer:
**PCT/EP1994/001394**

(87) Internationale Veröffentlichungsnummer:
**WO 1994/025603 (10.11.1994 Gazette 1994/25)**

(54) **CHOLESTERINOXIDASE AUS BREVIBACTERIUM STEROLICUM**

CHOLESTEROL-OXIDASE FROM BREVIBACTERIUM STEROLICUM

CHOLESTEROL-OXYDASE DU BREVIBACTERIUM STEROLICUM

(84) Benannte Vertragsstaaten:
**AT BE CH DE DK ES FR GB GR IE IT LI LU NL PT SE**

(30) Priorität: **05.05.1993 DE 4314793**
           **09.12.1993 DE 4342012**

(43) Veröffentlichungstag der Anmeldung:
**28.02.1996 Patentblatt 1996/09**

(73) Patentinhaber: **Roche Diagnostics GmbH**
     **68305 Mannheim (DE)**

(72) Erfinder: **JARSCH, Michael**
     **D-83670 Bad Heilbrunn (DE)**

(56) Entgegenhaltungen:
     **EP-A- 0 452 112          EP-A- 0 560 983**

• **GENE. Bd. 103 , 1991 , AMSTERDAM NL Seiten 93 - 96 T. OHTA ET AL 'Sequence of gene choB encoding cholesterol oxidase of Brevibacterium sterolicum: comparison with choA of Streptomyces sp. SA-COO' in der Anmeldung erwähnt**
• **BIOSCIENCE, BIOTECHNOLOGY, AND BIOCHEMISTRY Bd. 56, Nr. 11 , November 1992 Seiten 1786 - 1791 T. OHTA ET AL 'Hyperexpression and analysis of choB encoding cholesterol oxidase of Brevibacterium sterolicum in Escherichia coli and Streptomyces lividans' in der Anmeldung erwähnt**

## Beschreibung

Die Erfindung betrifft eine Cholesterinoxidase aus Brevibacterium sterolicum, ein Verfahren zur Herstellung einer rekombinanten Cholesterinoxidase aus Brevibacterium sterolicum, eine für dieses Verfahren geeignete DNA-Sequenz, welche eine zytoplasmatische Expression der rekombinanten Cholesterinoxidase im Wirtsbakterium bewirkt, sowie die so erhältliche rekombinante Cholesterinoxidase.

Für die enzymatische Bestimmung von Cholesterin ist die Cholesterinoxidase von großer Bedeutung. Sie katalysiert die Oxidation von Cholesterin zu Cholesten-3-on und $H_2O_2$. Cholesterinoxidase aus verschiedenen Organismen wie Pseudomonas, Mycobacterium, Nocardia, Arthrobacter und Brevibacterium sind bereits beschrieben worden (T. Uwajima et al., Agr. Biol. Chem. 37 (1973), 2345 - 2350). Alle diese bekannten Cholesterinoxidasen sind sezernierte Proteine. Das Bodenbakterium Brevibacterium sterolicum KY 3643 (ATCC 21387) zeigt eine besonders hohe Aktivität der Cholesterinoxidase. Aus diesem Bakterium sind drei Isoenzyme der Cholesterinoxidase bekannt, die sich in ihrem isoelektrischen Punkt, der Substratspezifität gegenüber verschiedenen Steroiden, der Affinität gegenüber Cholesterin im pH-Optimum und der DNA bzw. Aminosäuresequenz unterscheiden (EP-A 0 452 112 und EP-A 560 983). Die Cholesterinoxidase I aus Brevibacterium sterolicum zeigt eine geringe Affinität zu Cholesterin ($K_M$ 1,1 x $10^{-3}$ mol/l) und ist aus Brevibacterium sterolicum nur in geringer Ausbeute erhältlich. Die Expression einer kompletten für die Cholesterinoxidase I kodierenden DNA in E. coli wurde bereits versucht, ist jedoch bislang nicht gelungen (K. Fujishiro et al., Biochem. Biophys. Res. Com. 172 (1990), 721 - 727, T. Ohta et al., Gene 103 (1991), 93 - 96). Auch die Expression spezieller Deletionsmutanten der für die Cholesterinoxidase I kodierenden DNA, welche mit Teilen des lac z Gens fusioniert wurden, führte zu keiner befriedigenden Expression in E. coli (T. Ohta et al., Biosci. Biotech. Biochem. 56 (1992), 1786 - 1791). In der EP-A 0 452 112 wird die Klonierung und Expression von weiteren Cholesterinoxidasen aus Brevibacterium sterolicum beschrieben. Die Expression dieser DNAs führt jedoch ebenfalls nicht zu einer ausreichenden Menge an aktiver Cholesterinoxidase.

Aufgabe der Erfindung war es, eine Cholesterinoxidase mit hoher Affinität zu Cholesterin in großen Mengen und in aktiver Form zur Verfügung zu stellen.

Diese Aufgabe wird gelöst durch eine Cholesterinoxidase, welche die in SEQ ID NO 2 gezeigte Aminosäuresequenz aufweist. Diese Cholesterinoxidase ist aus Brevibacterium sterolicum erhältlich oder auch rekombinant herstellbar.

Es hat sich überraschenderweise gezeigt, daß eine derartige Cholesterinoxidase rekombinant in großer Menge und in aktiver Form hergestellt werden kann. Diese Cholesterinoxidase weist ein Molekulargewicht von 60 kD, einen isoelektrischen Punkt von ca. 5,5 (jeweils gemessen im Phast-System, Pharmacia-LKB) sowie einen $K_M$-Wert für Cholesterin von 1 x $10^{-4}$ mol/l (in 0,5 mol/l Kaliumphosphatpuffer pH 7,5 bei 25°C) auf und ist in einem pH-Bereich von 5,5 bis 8,0 wirksam.

Es hat sich gezeigt, daß diese Cholesterinoxidase in großer Menge in aktiver Form erhalten werden kann, wenn für eine heterologe Expression eine DNA verwendet wird, welche für ein Peptid mit Cholesterinoxidase-Aktivität kodiert mit der in SEQ ID NO 1 gezeigten DNA-Sequenz oder der dazu komplementären DNA-Sequenz.

Vorzugsweise wird eine DNA verwendet, welche die in SEQ ID NO 1 gezeigte Sequenz aufweist. In dem Fachmann geläufiger Weise können jedoch degenerierte Codons durch andere Codons, welche für die gleiche Aminosäure kodieren, ersetzt werden. Zusätzlich soll die verwendete DNA eine der in SEQ ID NO 3, 4 und/oder 5 gezeigten DNA-Sequenzen aufweisen und für ein Peptid mit Cholesterinoxidase-Aktivität kodieren. Unter einem Peptid mit Cholesterinoxidase-Aktivität ist ein solches Peptid zu verstehen, welches die Oxidation von Cholesterin (5-Cholesten-3-β-ol) zu 4-Cholesten-3-on und $H_2O_2$ katalysiert.

Ein weiterer Gegenstand der Erfindung ist daher eine DNA, welche für ein Peptid mit Cholesterinoxidase-Aktivität kodiert mit der in SEQ ID NO 1 gezeigten DNA-Sequenz oder der dazu komplementären DNA-Sequenz.

Mit einer solchen DNA kann eine mindestens 10fach höhere Aktivität der rekombinant hergestellten Cholesterinoxidase im Rohextrakt erhalten werden als mit den bislang beschriebenen Verfahren und Cholesterinoxidasen.

Ein weiterer Gegenstand der Erfindung ist ein Verfahren zur Herstellung einer rekombinanten Cholesterinoxidase durch Transformation einer geeigneten Wirtszelle mit einer erfindungsgemäßen DNA, welche in einem geeigneten Expressionssystem vorliegt, Kultivierung der transformierten Wirtszellen und Isolierung der gebildeten Cholesterinoxidase aus dem Zytoplasma der transformierten Zellen.

Mit diesem Verfahren ist es überraschenderweise möglich, eine rekombinante Cholesterinoxidase in großer Menge und aktiver Form aus dem Zytoplasma der transformierten Wirtszelle zu erhalten. Dabei kann die verwendete DNA am 5'-Ende eine zusätzliche Nukleotidsequenz enthalten, die ein Translations-Startcodon, jedoch kein Stopcodon aufweist, wobei diese zusätzliche Nukleotidsequenz nicht zu einer Leserasterverschiebung führt und keine für die Sekretion des gebildeten rekombinanten Enzyms funktionell aktive Signalsequenz darstellt. Die Länge dieser Nukleotidsequenz beträgt etwa 3 bis 90 Basenpaare.

Vorzugsweise weist die zusätzliche Nukleotidsequenz eine der in den Sequenzprotokollen 6, 8, 10, 12, 14 und 16 gezeigten Sequenzen anstelle der nativen Signalsequenz auf.

Ein bevorzugter Gegenstand der Erfindung ist daher ein Verfahren zur Herstellung einer rekombinanten Cholesterinoxidase, wobei eine erfindungsgemäße DNA verwendet wird, welche am 5'-Ende eine der in SEQ ID NO 6, 8, 10, 12, 14 oder 16 gezeigten Sequenzen aufweist.

Die Transformation der für die rekombinante Herstellung verwendeten Wirtszellen erfolgt nach bekannten Verfahren (siehe z.B. Sambrook, Fritsch und Maniatis, "Molecular Cloning, A Laboratory Manual", Cold Spring Harbor Laboratory Press, Cold Spring Harbor 1989). Die transformierten Wirtszellen werden dann unter Bedingungen kultiviert, die eine Expression des Cholesterinoxidase-Gens erlauben. Je nach dem verwendeten Expressionsvektor ist hierfür in bekannter Weise gegebenenfalls die Zugabe eines Induktors (z.B. Lactose oder Isopropyl-β-D-thiogalactopyranosid (IPTG)) zum Kulturmedium, eine Temperaturerhöhung oder eine limitierte Glucosezufuhr zweckmäßig. Die Isolierung der rekombinanten Cholesterinoxidase aus dem Zytoplasma der transformierten Zellen erfolgt dann nach bekannten Verfahren.

Mit diesem Verfahren ist es möglich, die erfindungsgemäße Cholesterinoxidase als rekombinantes Enzym in einer Ausbeute von 8 - 20 U/ml zu erhalten. Die Expression des vollständigen Cholesterinoxidase-Gens, welches die Signalsequenz enthält, ergibt dagegen lediglich eine Ausbeute von unter 0,1 U/ml.

Ein bevorzugter Gegenstand der Erfindung ist eine erfindungsgemäße, für ein Peptid mit Cholesterinoxidase-Aktivität kodierende DNA, welche am 5'-Ende eine der in SEQ ID NO 6, 8, 10, 12, 14 und 16 gezeigten Sequenzen aufweist. Besonders bevorzugt sind die in den Sequenzprotokollen 18, 20, 22, 24, 26 und 29 gezeigten Sequenzen. Vorzugsweise liegen diese erfindungsgemäßen DNA-Sequenzen in einem Expressionsvektor kloniert vor. Mit Hilfe dieser DNA kann die erfindungsgemäße Cholesterinoxidase in beliebigen Mengen in den für die rekombinante Herstellung von Proteinen üblicherweise verwendeten Bakterien gewonnen werden. Vorzugsweise erfolgt die Expression in E. coli.

Ein weiterer Gegenstand der Erfindung ist daher eine rekombinante Cholesterinoxidase, welche von einer erfindungsgemäßen DNA kodiert wird und am N-terminalen Ende eine der in SEQ ID NO 7, 9, 11, 13, 15 oder 17 gezeigten Aminosäuresequenzen aufweist.

Diese rekombinante Cholesterinoxidase ist für einen enzymatischen Test zur Bestimmung von Cholesterin ebenso geeignet wie die übrigen aus dem Stand der Technik bekannten Cholesterinoxidasen. Falls erforderlich können in dem Fachmann geläufiger Weise durch in-vitro-Mutagenese zwischen diesen N-terminalen Sequenzen und der Aminosäuresequenz der reifen Cholesterin-oxidase Erkennungssequenzen für spezifische Proteasen wie z.B. der IgA-Protease, der Enterokinase oder des Faktors Xa integriert werden, so daß auch nach der zytoplasmatischen Expression der um diese N-terminalen Sequenzen verlängerten Cholesterinoxidase eine Abspaltung solcher anfusionierter N-terminaler Sequenzen möglich ist.

Ein bevorzugter Gegenstand der Erfindung ist eine rekombinante Cholesterinoxidase, welche die in SEQ ID NO 21, 23, 25, 27 oder 29 gezeigte Aminosäuresequenz aufweist, sowie die Verwendung einer solchen rekombinanten Cholesterinoxidase in einem enzymatischen Test zum Nachweis von Cholesterin. Dabei wird vorzugsweise das in der Cholesterinoxidasereaktion gebildete $H_2O_2$ in einer nachgeschalteten Indikatorreaktion als Maß für das in der Probe vorhandene Cholesterin bestimmt.

Die in den Beispielen genannten Plasmide pUC-Chol-B2-BB (DSM 8274), pmgl-SphI (DSM 8272) und pfl-20AT1-SD (DSM 8273) wurden am 05.05.1993 bei der Deutschen Sammlung für Zellkulturen und Mikroorganismen GmbH, Mascheroder Weg 1b, D - 3300 Braunschweig hinterlegt.

Die Anmeldung wird durch die folgenden Beispiele in Verbindungen mit den Sequenzprotokollen und Figuren näher erläutert.

| | |
|---|---|
| SEQ ID NO 1 | zeigt die Nukleinsäuresequenz der erfindungsgemäßen Cholesterinoxidase. |
| SEQ ID NO 2 | zeigt die Aminosäuresequenz der erfindungsgemäßen Cholesterinoxidase. |
| SEQ ID NO 3 - 5 | zeigen Nukleotidsequenzen aus erfindungsgemäßen, für ein Peptid mit Cholesterinoxidase-Aktivität kodierenden DNA's. |
| SEQ ID NO 6 - 17 | zeigen die N-terminalen Sequenzen der erfindungsgemäßen rekombinanten Cholesterinoxidasegene (SEQ ID NO 6, 8, 10, 12, 14 und 16) bzw. der dazugehörigen N-terminalen Aminosäuresequenzen (SEQ ID NO 7, 9, 11, 13, 15 und 17). |
| SEQ ID NO 18 - 29 | zeigen die Nukleinsäuresequenzen und dazugehörigen Aminosäuresequenzen von erfindungsgemäßen rekombinanten Cholesterinoxidasen. |

Dabei bedeuten:

| Signalsequenz | vollständige Sequenz | Konstrukt |
|---|---|---|
| SEQ ID NO 6-7 | SEQ ID NO 18-19 | plac-Chol-cyt |
| SEQ ID NO 8-9 | SEQ ID NO 20-21 | ppfl-Chol-cyt |
| SEQ ID NO 10-11 | SEQ ID NO 22-23 | ppfl-MSN3H-Chol-cyt |
| SEQ ID NO 12-13 | SEQ ID NO 24-25 | ppfl-MSN4H-Chol-cyt |
| SEQ ID NO 14-15 | SEQ ID NO 26-27 | ppfl-MSN4R2K-Chol-cyt |
| SEQ ID NO 16-17 | SEQ ID NO 28-29 | ppfl-MVM3H-Chol-cyt |

SEQ ID NO 30 - 33     zeigen vier Oligonukleotide für die Amplifikation eines Fragments des erfindungsgemäßen Cholesterinoxidase-Gens.

SEQ ID NO 34     zeigt die Sequenz eines Adapteroligonukleotids für die in vitro-Mutagenese des Cholesterinoxidase-Gens gemäß Beispiel 5.

Fig. 1     zeigt das Plasmid pUC-Chol-B2-BB.

Fig. 2     zeigt das Plasmid plac-Chol-cyt.

Fig. 3     zeigt das Plasmid ppfl-Chol-cyt.

Fig. 4     zeigt das Plasmid ppfl-MSN3H-Chol-cyt.

**Beispiel 1**

**Klonierung des Gens für Cholesterinoxidase aus Brevibacterium sterolicum**

Brevibacterium sterolicum (BMTU 2407) wird in 500 ml "nutrient broth" (Difco) 20 h bei 30°C angezüchtet. Die Zellen werden durch Zentrifugation geerntet. Die so gewonnene Zellmasse wird in 20 mmol/l Tris/HCl pH 8,0 zu 0,4 g Zell-Naßgewicht/ml resuspendiert. 2,5 ml dieser Suspension werden mit 5 ml 24 % (w/v) Polyethylenglycol 6000, 2,5 ml 20 mmol/l Tris/HCl pH 8,0 und 10 mg Lysozym versetzt und 14 h bei 4°C inkubiert. Dann erfolgt die Lyse der Zellen durch Zugabe von 1 ml 20 % (w/v) SDS und 2 mg Protease K und Inkubation für 1 h bei 37°C. Diese Lösung wird mit dem gleichen Volumen 20 mmol/l Tris/HCl pH 8,0 versetzt und dann pro ml 1 g CsCl sowie 0,8 mg Ethidiumbromid zugegeben. Diese Lösung wird durch Ultrazentrifugation 24 h bei 40.000 Upm in einem TV850 Vertikal-Rotor (DuPont) aufgetrennt. Die DNA-Bande wird dann mit einer Injektionsspritze-abgezogen. Die Entfernung des Ethidiumbromids und Ethanol-Fällung der DNA erfolgt wie bei Sambrook et al., Molecular Cloning, A Laboratory Manual (1989) beschrieben.

7 $\mu$g der so gewonnenen DNA werden partiell mit der Restriktionsendonuklease NlaIII (New England Biolab) geschnitten, auf einem 0,8 % Agarosegel elektrophoretisch aufgetrennt und ein Größenbereich von ca. 2 - 12 kb ausgeschnitten. Die DNA-Fragmente werden aus dem Gel isoliert, mit SphI geschnitten und anschließend in einen mit alkalischer Phosphatase aus Kälberdärm behandelten Plasmidvektor pUC19 ligiert. Dieser Ligationsansatz wird in kompetente E. coli K12 XL1-blue (Stratagene, Katalog-Nr. 200268) transformiert. Die transformierten Zellen werden auf Agarplatten mit LB-Medium, das 100 $\mu$g/ml Ampicillin enthält, ausplattiert und über Nacht bei 37°C inkubiert. Die hochgewachsenen Kolonien werden auf Nitrocellulosefilter (Schleicher und Schüll) übertragen, durch Behandlung mit Toluol/Chloroform-Dampf lysiert und die Filter mit der Kolonieseite auf Indikatorplatten (s.u.) übertragen. Auf diesen Indikatorplatten erfolgt der Nachweis auf eine Cholesterinoxidase-Aktivität durch 15- bis 30-minütige Inkubation bei Raumtemperatur.

Klone, die eine Farbreaktion zeigen, werden ausgewählt und isoliert. Zur Kontrolle werden diese E. coli-Klone auf einer Agarplatte mit LB-Medium, das 100 $\mu$g/ml Ampicillin enthält, ausgestrichen, über Nacht bei 37°C inkubiert, die angewachsenen Kolonien zur Verifizierung nochmals auf zwei verschiedene Nitrozellulosefilter transferiert und wie oben beschrieben mit Toluol/Chloroformdampf aufgeschlossen. Ein Filter wird wieder auf eine der oben beschriebenen Indikatorplatten aufgelegt, der andere Falter auf eine Indikatorplatte ohne Cholesterin. Eine positive Farbreaktion zeigt sich nur auf den kompletten Indikatorplatten mit dem Substrat Cholesterin. Damit wird nachgewiesen, daß die durch den entsprechenden E. coli-Klon hervorgerufene Farbreaktion tatsächlich durch aktive Cholesterinoxidase verursacht wird.

**Herstellung der Indikatorplatten:**

Für den Plattentest zur Bestimmung von Cholesterinoxidase-Aktivität werden 100 ml 2%ige low-melting-point-Agarose (Sea Plaque BIOzym 50113) aufgeschmolzen und bei einer Temperatur von 42°C eine vorgewärmte Lösung von:

- 48 mg 4-Aminoantipyrin (Boehringer Mannheim GmbH, Katalog-Nr. 073474)
- 306 mg EST (N-Ethyl-N-sulfoethyl-3-methylanilinkaliumsalz (Boehringer Mannheim GmbH, Katalog-Nr. 586854))
- 2,5 mg Meerrettichperoxidase Reinheitsgrad II (ca. 260 U/mg (Boehringer Mannheim GmbH, Katalog-Nr. 005096))
- 60 $\mu$l Natriumazidlösung (20%ig)
- 10 ml 1 mol/l Kaliumphosphat pH 7,2
- 150 mg Cholsäurenatriumsalz (Merck, Katalog-Nr. 12448)
- 10 ml Cholesterinsubstratlösung (s. u.)
- $H_2O$ ad 100 ml

zu der aufgeschmolzenen Agarose gegeben, vorsichtig gemischt, jeweils 10 ml in Petrischalen gegossen und zur Aufbewahrung dunkel gehalten.

**Cholesterinsubstratlösung:**

500 mg Cholesterin (Boehringer Mannheim GmbH, Katalog-Nr. 121312) werden in 12,5 ml 1-Propanol (Merck, Katalog-Nr. 997) gelöst, nach Zugabe von 10 g Thesit (Boehringer Mannheim GmbH, Katalog-Nr. 006190) gut gemischt und $H_2O$ ad 100 ml zugegeben. Die Substratlösung kann bei Raumtemperatur mehrere Monate aufbewahrt werden.

**Beispiel 2**

**Charakterisierung des Cholesterinoxidase-Gens**

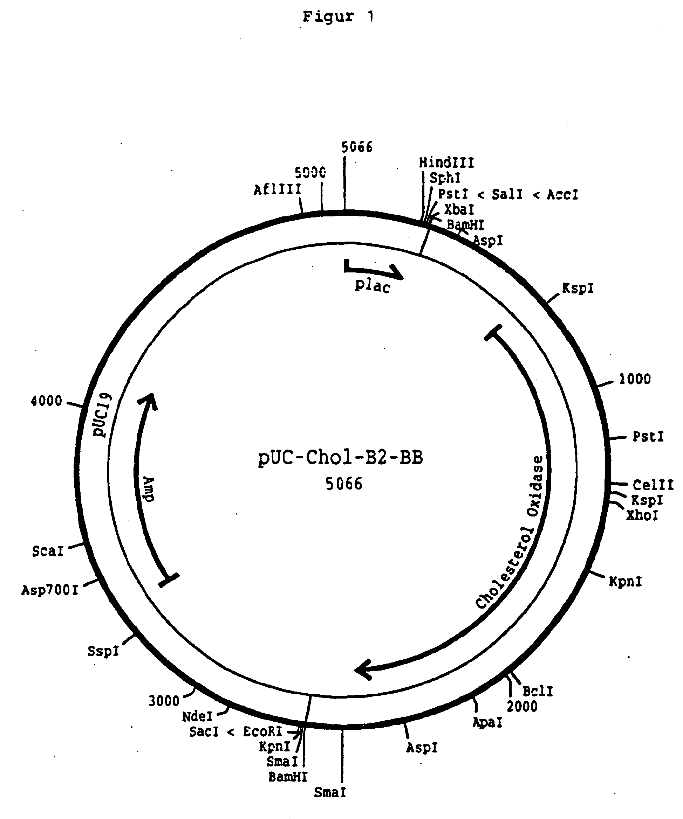

Das Plasmid eines gemäß Beispiel 1 erhaltenen Klons (pUC-Chol-B2) wird nach Standardmethoden isoliert und einer Restriktionskartierung mit den Restriktionsendonukleasen BamHI, EcoRI, KpnI, XhoI, PstI unterzogen. Es zeigt sich, daß ein DNA-Fragment aus dem Genom von Brevibacterium in der Größe von ca. 5,5 kb in dem Plasmid pUC-Chol-B2 insertiert ist. Durch Subklonierung verschiedener Teilfragmente dieses 5,5 kb-Stückes und anschließender Bestimmung der Cholesterin-oxidase-Aktivität der erhaltenen E. coli-Klone kann das Cholesterinoxidase-Gen auf ein BamHI-Fragment von 2,3 kb-Größe eingeengt werden. Das Plasmid mit diesem Fragment wird pUC-Chol-B2-BB genannt (Fig. 1). Die DNA-Sequenz dieses Fragmentes wird bestimmt und auf einem Leseraster, das für Cholesterinoxidase kodiert, hin untersucht. Die Sequenz dieses Leserahmens für die reife Cholesterinoxidase ist in SEQ ID NO 1 wiedergegeben.

**Beispiel 3**

**Konstruktion eines Plasmids zur Expression des Cholesterinoxidase-Gens mit heterologer Signalsequenz**

Ein Vergleich der N-terminalen Aminosäuresequenz von Cholesterinoxidase, die aus Brevibacterium isoliert wurde, mit dem gesamten für Cholesterinoxidase kodierenden Leseraster von pUC-Chol-B2-BB zeigt, daß im reifen Protein die ersten 52 kodierten Aminosäuren der Gensequenz fehlen. Diese 52 Aminosäuren zeigen die Struktur einer typischen Exportsignalsequenz gram-positiver Prokaryonten (von Heijne, Biochim. Biophys. Acta 947 (1988), 307 - 333). Für die Konstruktion von rekombinanten Cholesterinoxidase-Genen, bei denen diese Signalsequenz gegen andere Sequenzen ersetzt ist, wird zunächst ein 387 bp großes DNA-Fragment aus dem Plasmid pUC-Chol-B2-BB unter Verwendung der in SEQ ID NO 30 und 31 gezeigten Oligonukleotide mittels PCR amplifiziert. Dieses Fragment enthält den für den N-terminalen Teil der reifen Oxidase kodierenden Bereich mit einer neuen SphI-Schnittstelle direkt vor dem N-Terminus der Aminosäuresequenz des reifen Enzyms. Dieses PCR-Fragment wird mit SphI und PstI gespalten und zusammen mit einem PstI EcoRI-Fragment aus pUC-Chol-B2-BB, das den restlichen Anteil des Cholesterinoxidase-Gens enthält, in den mit SphI und EcoRI gespaltenen Expressionsvektor pmglSphI ligiert und so der Vektor pmgl-Chol-SB erhalten. In diesem Vektor enthält das Cholesterinoxidase-Gen eine in E. coli funktionelle Signalsequenz aus Salmonella typhimurium (beschrieben in WO 88/093773).

**Beispiel 4**

**Konstruktion eines Plasmids zur Expression des Cholesterin-oxidase-Gens ohne Signalpeptid-kodierende Sequenz unter Kontrolle des lacUV5-Promotors**

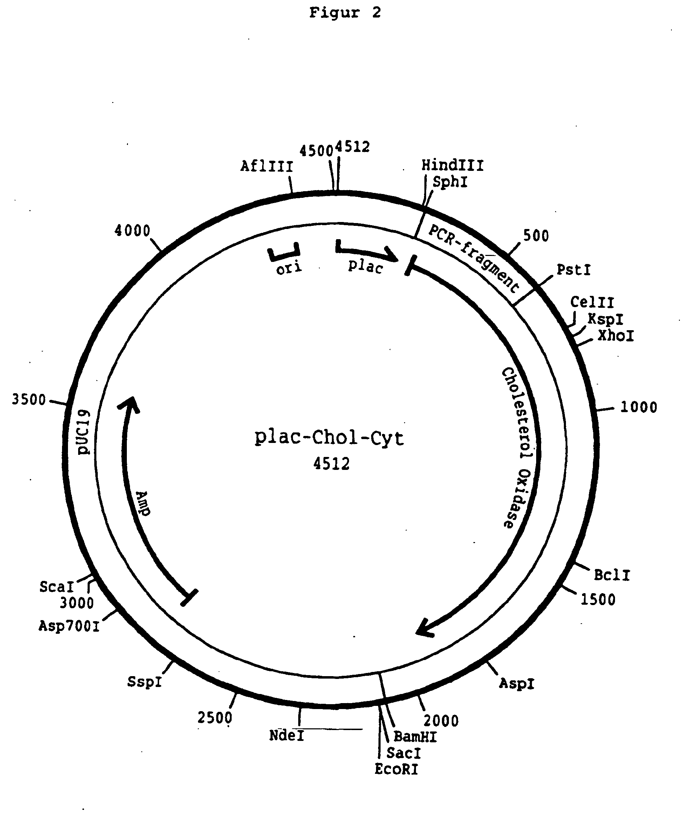

Aus dem Plasmid pmgl-Chol-SB wird durch Behandlung mit den Restriktionsendonukleasen SphI und BamBI ein DNA-Fragment von ca. 1,85 kb Größe herausgeschnitten, das den gesamten Anteil der kodierenden Sequenz der reifen Cholesterinoxidase, aber nicht die für das Signal-Peptid kodierende Sequenz enthält. Dieses Fragment wird in den vorher mit SphI und BamBI geschnittenen Plasmidvektor pUC19 eingesetzt. In dem so erhaltenen Plasmid plac-Chol-cyt liegt das Cholesterin-oxidase-Gen im korrekten Leseraster an die ersten zehn Codons des lacZ'-Gens aus pUC19 anfusioniert vor und liegt unter der Kontrolle des lacUV5-Promotors (Fig. 2).

**Beispiel 5**

**Konstruktion eines Plasmids zur Expression des Cholesterin-oxidase-Gens ohne Signalpeptid-kodierende Sequenz unter Kontrolle des sauerstoffregulierten pfl-Promotors**

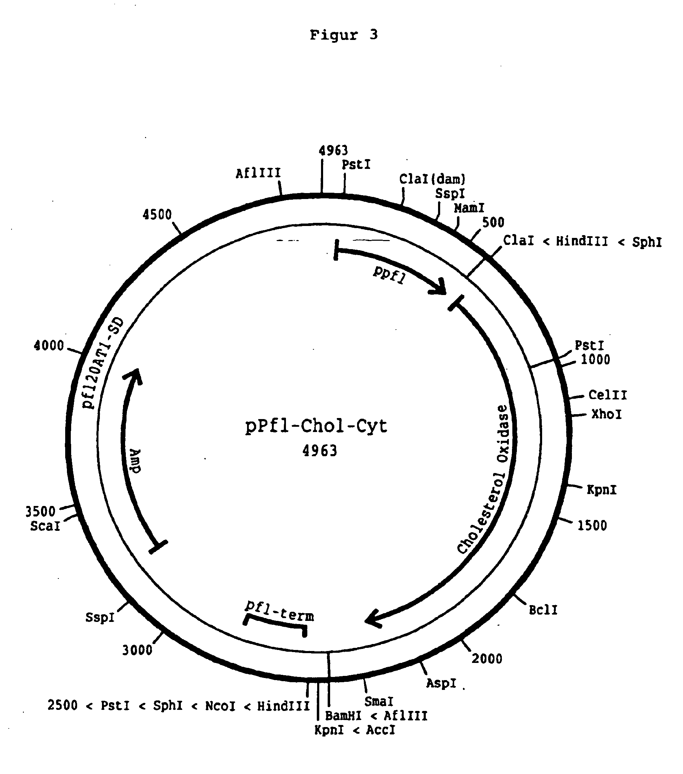

Durch PCR-Technik wird aus dem Plasmid plac_Chol_cyt unter Verwendung der in SEQ ID NO 32 und 33 dargestellten Oligonukleotide ein DNA-Fragment von 432 bp Größe erzeugt, das vor dem ATG-Startcodon eine ClaI-Schnittstelle enthält. Dieses PCR-Fragment wird mit ClaI und PstI geschnitten. Durch Behandlung mit den Restriktionsendonukleasen PstI und BamHI wird aus dem Plasmid plac-Chol-cyt weiterhin ein Fragment mit dem restlichen C-terminalen Anteil des Cholesterinoxidase-Gens herausgeschnitten. Beide Fragmente werden simultan in den mit BamHI und ClaI gespaltenen Expressionsvektor pfl 20AT1-SD einligiert. Das korrekte Ligationsprodukt enthält nun den Leserahmen der reifen Cholesterinoxidase anfusioniert an die ersten zehn Codons des lacZ'-Gens aus pUC19 unter der Kontrolle des sauerstoffregulierten pfl-Promotors (Fig. 3). Dieses Plasmid trägt die Bezeichnung ppfl-Chol-cyt.

**Beispiel 6**

**Konstruktion eines Plasmids zur Expression des Cholesterin-oxidase-Gens mit alternativer N-terminaler Fusionssequenz**

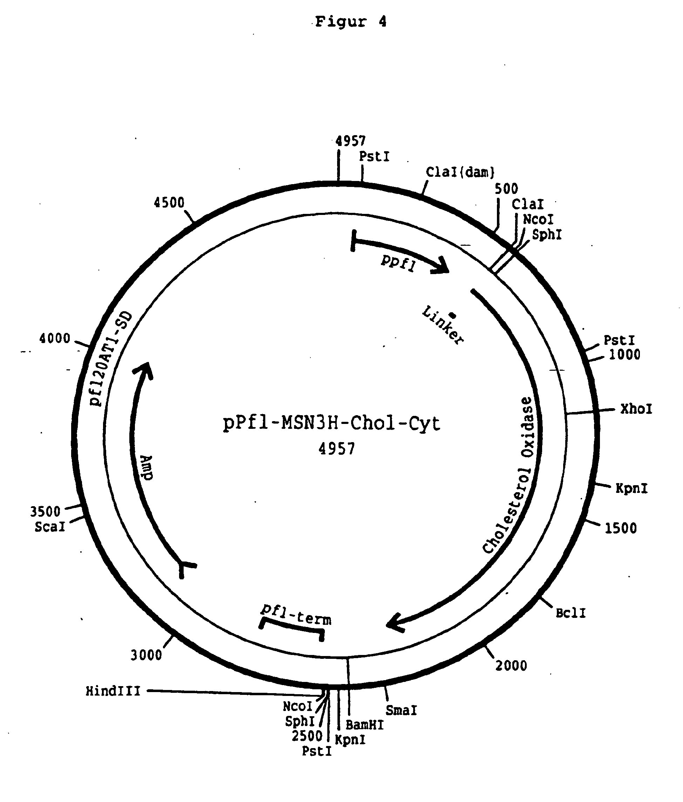

Zur Entfernung der im 3' untranslatierten Bereich des Cholesterinoxidase-Gens gelegenen SphI-Schnittstelle des Plasmids ppfl-Chol-cyt wird die Plasmid-DNA mit SmaI und EcoRV geschnitten und wieder religiert. 100 ng des so entstandenen Plasmids ppfl-Chol-cyt-Aterm werden dann mit den Restriktionsenzymen ClaI und SphI gespalten. Das entstandende 4,76 kb große DNA-Fragment wird in low-melting-point Agarose elektrophoretisch aufgetrennt, ausgeschnitten und eluiert (Glassmilk®-Kit, Bio 101). 100ng des so gereinigten DNA-Fragments werden mit 50 pmol eines Adapter-Oligonukleotids mit der in SEQ ID NO 34 dargestellten Sequenz (wobei "N" eine äquimolare Mischung aller 4 Basen bedeutet) versetzt und 2 Stunden bei 37°C mit T4-DNA-Ligase behandelt. Anschließend wird der Ansatz mit einer Mischung aus 4 dNTP's (Endkonz. 0,125 mmol/l) versetzt und 40 Minuten bei 37°C mit Klenow-DNA-Polymerase behandelt. Die so erhaltene Plasmid-DNA wird in E. coli XL1-blue (Stratagene) transformiert. Mit Hilfe des in Beispiel 1 beschriebenen Kolonie-Aktivitätstest werden einzelne Kolonien von erhaltenen Klonen bezüglich ihrer Cholesterinoxidase-Aktivität verglichen. Klone mit hoher Cholesterinoxidase-Aktivität werden isoliert und die Plasmid-DNA durch Restriktionsanalvse und DNA-Sequenzierung charakterisiert. Für das Plasmid eines Klons mit besonders hoher Cholesterinoxidase-Aktivität wird die Sequenz SEQ ID NO 23 ermittelt. Das betreffende Plasmid wird ppfl-MSM3H-Chol-cyt-Aterm genannt. Es ist zu_erwarten, daß in der dargestellten Art und Weise nach Isolierung und Charakterisierung genügend vieler verschiedener Klone auch noch weitere für eine besonders hohe Expression geeignete Klone gefunden werden können. Zur Wiedervervollständigung des 3'-untranslatierten Anteils wird das Plasmid ppfl-MSN3H-Chol-cyt-Aterm mit ClaI und XhoI geschnitten. Ein DNA-Fragment von ca. 1,1kb mit der Translationsinitiationsregion und dem N-terminalen Anteil des Cholesterinoxidase-Gens wird isoliert und in das ebenfalls mit ClaI und XhoI geschnittene Plasmid ppfl-Chol-cyt einligiert (Fig. 4). Das erhaltene Plasmid trägt die Bezeichnung ppfl-MSN3H-Chol-cyt.

**Beispiel 7**

**Vergleich der Bildung von Cholesterinoxidase durch die verschiedenen Expressionsplasmide in E. coli**

Die Plasmide pUC-Chol-B2, pUC-Chol-B2-BB, pmgl-Chol-SB, plac-Chol-cyt, ppfl-Chol-cyt, ppfl-MSN3H-Chol-cyt werden jeweils in E. coli K12 XL1-blue transformiert. Zum Vergleich der gebildeten Enzymmenge werden die Klone jeweils 15 Stunden bei 30°C in LB-Medium, das 200 μg/ml Ampicillin und folgende weiteren Zusätze enthält, angezogen:

Klone mit den Plasmiden pUC-Chol-B2, pUC-Chol-B2-BB, plac-Chol-cyt, bei denen das Cholesterinoxidase-Gen jeweils unter der Kontrolle des lacUV5-Promotors steht, bekommen zusätzlich 1 mmol/l IPTG, der Klon mit dem Plasmid pmgl-Chol-SB mit dem Glucose-reprimierten mgl-Promotor erhält keinen weiteren Zusatz, Klone mit den Plasmiden ppfl-Chol-cyt, ppfl-MSN3H-Chol-cyt mit dem sauerstoffregulierten pfl-Promotor erhalten 0,4% Glucose und werden in Stickstoff begasten verschlossenen Serumflaschen angezogen, wobei das Medium mit KOH auf pH 7,0 eingestellt wurde. Nach erfolgter Anzucht wird die erreichte Zelldichte durch photometrische Messung der Trübung bei 420 nm bestimmt. Die Zellen von 1 ml Kulturbrühe werden dann durch Zentrifugation in einer Mikrozentrifuge bei 10.000 g sedimentiert und wieder in 0,5 ml $H_2O$ bidest resuspendiert. Der Zellaufschluß erfolgt durch 2 x 30 Sekunden Ultraschallbehandlung (Branson Sonfier, Modell 450, Standard-Microtip, Konisch). Die so erhaltenen Zellextrakte werden nach entsprechender Verdünnung in den folgenden Enzymtest eingesetzt: Hierzu werden in Quartz-Küvetten pipettiert: 3 ml Kaliumphosphatpuffer (0,5 mol/l, pH 7,5), der 0,4 % Thesit® (Boehringer Mannheim GmbH, Katalog-Nr. 006190) enthält,

0,1 ml Cholesterinlösung (0,4 % Cholesterin, 10 % 1-Propanol, 10 % Thesit®),

0,02 ml $H_2O_2$ (0,49 mol/l in bidest. Wasser),

es wird gemischt, nach Zugabe von 0,02 ml Katalase (aus Rinderleber, 20 mg Protein/ml, spezifische Aktivität ca. 65.000 U/mg, Boehringer Mannheim GmbH, Katalog-Nr. 0156744 unmittelbar vor Messung mit eiskaltem Kaliumphosphatpuffer, der 0,4 % Thesit enthält, auf 0,075 - 0,15 U/ml verdünnt) erneut gemischt, die Lösung auf eine Temperatur von 25°C gebracht und anschließend die Reaktion durch Zugabe von 0,05 ml Probelösung gestartet. Nach vorsichtigem Mischen wird die Absorptionsänderung bei 240 nm verfolgt und die Aktivität der Cholesterinoxidase aus dem linearen Bereich der Absorptionskurve ermittelt:

$$\text{Aktivität} = \frac{3{,}19}{\in 240 \times 0{,}05 \times 1} \ \Delta\ A\ \text{min. (U/ml Probelösung)}$$

wobei $\in 240 = 15{,}5 \ mmol^{-1} \times 1 \times cm^{-1}$ ist.

Die erhaltenen Werte für Zelldichte und Enzymaktivität sind in Tabelle 1 dargestellt.

Tabelle 1

| Klon/Plasmid | Zelldichte (E 420) | Units je Zelldichte | Units pro ml |
|---|---|---|---|
| pUC-Chol-B2 | 7,0 | 0,007 | 0,049 |
| pUC-Chol-B2-BB | 8,4 | 0,068 | 0,571 |
| pmgl-Chol-SB | 1,3 | 0,014 | 0,018 |
| plac-Chol-cyt | 8,6 | 0,725 | 6,235 |
| ppfl-Chol-cyt | 1,25 | 1,675 | 2,094 |
| ppfl-MSN3H-Chol-cyt | 3,7 | 1,463 | 5,413 |

Die erhaltenen Ergebnisse zeigen, daß mit solchen Konstrukten, die eine zytoplasmatische Expression der Cholesterinoxidase bewirken, eine deutlich höhere Aktivität der rekombinant hergestellten Cholesterinoxidase erhalten werden kann als mit solchen Konstrukten, die zu einer Sekretion der rekombinant hergestellten Cholesterinoxidase führen.

**Patentansprüche**

1. Aktive Cholesterinoxidase, **dadurch gekennzeichnet, daß** sie die in SEQ ID NO 2 gezeigte Aminosäuresequenz aufweist.

2. DNA, welche für ein Peptid mit Cholesterinoxidase-Aktivität kodiert mit der in SEQ ID NO 1 gezeigten DNA-Sequenz oder der dazu komplementären DNA-Sequenz.

3. Verfahren zur Herstellung einer rekombinanten Cholesterinoxidase durch Transformation einer geeigneten Wirtszelle mit einer DNA gemäß Anspruch 2, welche in einem geeigneten Expressionssystem kloniert vorliegt, Kultivierung der transformierten Wirtszellen und Isolierung der exprimierten Cholesterinoxidase aus dem Zytoplasma der transformierten Zellen.

4. Verfahren gemäß Anspruch 3, **dadurch gekennzeichnet, daß** die verwendeten DNA am 5'-Ende eine der in SEQ ID NO 6, 8, 10, 12, 14 oder 16 gezeigten Sequenzen aufweist.

5. DNA gemäß Anspruch 2, **dadurch gekennzeichnet, daß** sie am 5'-Ende eine der in SEQ ID NO 6, 8, 10, 12, 14 oder 16 gezeigten Sequenzen aufweist.

6. DNA gemäß Anspruch 5, **dadurch gekennzeichnet, daß** sie eine der in SEQ ID NO 18, 20, 22, 24, 26 oder 28 gezeigten Sequenzen aufweist.

7. Rekombinante Cholesterinoxidase, **dadurch gekennzeichnet, daß** sie von einer DNA gemäß Anspruch 2 kodiert wird und am N-terminalen Ende eine der in SEQ ID NO 7, 9, 11, 13, 15 oder 17 gezeigten Sequenzen aufweist.

8. Rekombinante Cholesterinoxidase gemäß Anspruch 7, **dadurch gekennzeichnet, daß** sie eine der in SEQ ID NO 21, 23, 25, 27 und 29 gezeigten Sequenzen aufweist.

9. Verwendung einer rekombinanten Cholesterinoxidase gemäß einem der Ansprüche 7 oder 8 in einem enzymatischen Test zur Bestimmung von Cholesterin.

**Claims**

1. Active cholesterol oxidase, **characterized in that** it has the amino acid sequence shown in SEQ ID NO 2.

2. DNA which codes for a peptide with cholesterol oxidase activity having the DNA sequence shown in SEQ ID NO 1 or the DNA sequence which is complementary thereto.

3. Process for the production of a recombinant cholesterol oxidase by transformation of a suitable host cell with a DNA as claimed in claim 2 which is present cloned in a suitable expression system, culturing the transformed host cells and isolating the expressed cholesterol oxidase from the cytoplasm of the transformed cells.

4. Process as claimed in claim 3, **characterized in that** the DNA used has one of the sequences shown in SEQ ID NO 6, 8, 10, 12, 14 or 16 at the 5' end.

5. DNA as claimed in claim 2, **characterized in that** it has one of the sequences shown in SEQ ID NO 6, 8, 10, 12, 14 or 16 at the 5' end.

6. DNA as claimed in claim 5, **characterized in that** it has one of the sequences shown in SEQ ID NO 18, 20, 22, 24, 26 or 28.

7. Recombinant cholesterol oxidase, **characterized in that** it is coded by a DNA as claimed in claim 2 and has one of the sequences shown in SEQ ID NO 7, 9, 11, 13, 15 or 17 at the N-terminal end.

8. Recombinant cholesterol oxidase as claimed in claim 7, **characterized in that** it has one of the sequences shown in SEQ ID NO 21, 23, 25, 27 or 29.

9. Use of a recombinant cholesterol oxidase as claimed in one of the claims 7 or 8 in an enzymatic test for the determination of cholesterol.

**Revendications**

1. Cholestérol oxydase active, **caractérisée en ce qu'**elle présente la séquence d'acides aminés représentée dans SEQ ID NO: 2.

2. ADN qui code pour un peptide possédant une activité de cholestérol oxydase comprenant la séquence d'ADN représentée dans SEQ ID NO: 1 ou la séquence d'ADN complémentaire à celle-ci.

3. Procédé pour la préparation d'une cholestérol oxydase recombinante par transformation d'une cellule hôte appropriée avec un ADN selon la revendication 2, qui est présent à l'état cloné dans un système d'expression approprié, par mise en culture des cellules hôtes transformées et par isolation de la cholestérol oxydase exprimée à partir du cytoplasme des cellules transformées.

4. Procédé selon la revendication 3, **caractérisé en ce que** l'ADN utilisé présente, à l'extrémité 5', une des séquences représentées dans SEQ ID NO: 6, 8, 10, 12, 14 ou 16.

5. ADN selon la revendication 2, **caractérisé en ce qu'**il présente, à son extrémité 5', une des séquences représentées dans SEQ ID NO: 6, 8, 10, 12, 14 ou 16.

6. ADN selon la revendication 5, **caractérisé en ce qu'**il présente une des séquences représentées dans SEQ ID NO: 18, 20, 22, 24, 26 ou 28.

7. Cholestérol oxydase recombinante, **caractérisée en ce qu'**elle est encodée par un ADN selon la revendication 2 et **en ce qu'**elle présente, à son extrémité amino terminale, une des séquences représentées dans SEQ ID NO: 7, 9, 11, 13, 15 ou 17.

8. Cholestérol oxydase recombinante selon la revendication 7, **caractérisé en ce qu'**elle présente une des séquences représentées dans SEQ ID NO: 21, 23, 25, 27 et 29.

9. Utilisation d'une cholestérol oxydase recombinante selon l'une quelconque des revendications 7 ou 8, dans un test enzymatique pour la détermination de cholestérol.

Figur 1

# Figur 2

plac-Chol-Cyt
4512

Figur 3

## Figur 4

Figur 4 — pPfl-MSN3H-Chol-Cyt, 4957

SEQUENZPROTOKOLL

(1) ALGEMEINE INFORMATION:

(i) ANMELDER:

(A) NAME: Boehringer Mannheim GmbH

(B) STRASSE: Sandhofer Str. 116
(C) ORT: Mannheim
(E) LAND: Deutschland
(F) POSTLEITZAHL: D - 6800

(ii) ANMELDETITEL: Cholesterinoxidase aus Brevibacterium sterolicum

(iii) ANZAHL DER SEQUENZEN: 34

(iv) COMPUTER-LESBARE FORM:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:

(A) LANGE: 1683 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:

(A) NAME/SCHLÜSSEL: CDS
(B) LAGE: 1..1683

(xi) SEQUENZBESCHREIBUNG: SEQ ID NO: 1:

```
TCG ACC GGG CCG GTC GCG CCG CTT CCG ACG CCG CCG AAC TTC CCG AAC        48
Ser Thr Gly Pro Val Ala Pro Leu Pro Thr Pro Pro Asn Phe Pro Asn
 1               5                   10                  15

GAC ATC GCG CTG TTC CAG CAG GCG TAC CAG AAC TGG TCC AAG GAG ATC        96
Asp Ile Ala Leu Phe Gln Gln Ala Tyr Gln Asn Trp Ser Lys Glu Ile
            20                  25                  30

ATG CTG GAC GCC ACT TGG GTC TGC TCG CCC AAG ACG CCG CAG GAT GTC       144
Met Leu Asp Ala Thr Trp Val Cys Ser Pro Lys Thr Pro Gln Asp Val
            35                  40                  45

GTT CGC CTT GCC AAC TGG GCG CAC GAG CAC GAC TAC AAG ATC CGC CCG       192
Val Arg Leu Ala Asn Trp Ala His Glu His Asp Tyr Lys Ile Arg Pro
        50                  55                  60

CGC GGC GCG ATG CAC GGC TGG ACC CCG CTC ACC GTG GAG AAG GGG GCC       240
Arg Gly Ala Met His Gly Trp Thr Pro Leu Thr Val Glu Lys Gly Ala
 65                 70                  75                  80

AAC GTC GAG AAG GTG ATC CTC GCC GAC ACG ATG ACG CAT CTG AAC GGC       288
Asn Val Glu Lys Val Ile Leu Ala Asp Thr Met Thr His Leu Asn Gly
                85                  90                  95

ATC ACG GTG AAC ACG GGC GGC CCC GTG GCT ACC GTC ACC GCC GGT GCC       336
Ile Thr Val Asn Thr Gly Gly Pro Val Ala Thr Val Thr Ala Gly Ala
            100                 105                 110

GGC GCC AGC ATC GAG GCG ATC GTC ACC GAA CTG CAG AAG CAC GAC CTC       384
Gly Ala Ser Ile Glu Ala Ile Val Thr Glu Leu Gln Lys His Asp Leu
            115                 120                 125

GGC TGG GCC AAC CTG CCC GCT CCG GGT GTG CTG TCG ATC GGT GGC GCC       432
Gly Trp Ala Asn Leu Pro Ala Pro Gly Val Leu Ser Ile Gly Gly Ala
            130                 135                 140

CTT GCG GTC AAC GCG CAC GGT GCG GCG CTG CCG GCC GTC GGC CAG ACC       480
Leu Ala Val Asn Ala His Gly Ala Ala Leu Pro Ala Val Gly Gln Thr
145                 150                 155                 160

ACG CTG CCC GGT CAC ACC TAC GGT TCG CTG AGC AAC CTG GTC ACC GAG       528
Thr Leu Pro Gly His Thr Tyr Gly Ser Leu Ser Asn Leu Val Thr Glu
                165                 170                 175

CTG ACC GCG GTC GTC TGG AAC GGC ACC ACC TAC GCA CTC GAG ACG TAC       576
Leu Thr Ala Val Val Trp Asn Gly Thr Thr Tyr Ala Leu Glu Thr Tyr
            180                 185                 190

CAG CGC AAC GAT CCT CGG ATC ACC CCA CTG CTC ACC AAC CTC GGG CGC       624
Gln Arg Asn Asp Pro Arg Ile Thr Pro Leu Leu Thr Asn Leu Gly Arg
            195                 200                 205

TGC TTC CTG ACC TCG GTG ACG ATG CAG GCC GGC CCC AAC TTC CGT CAG       672
Cys Phe Leu Thr Ser Val Thr Met Gln Ala Gly Pro Asn Phe Arg Gln
     210                 215                 220

CGG TGC CAG AGC TAC ACC GAC ATC CCG TGG CGG GAA CTG TTC GCG CCG       720
Arg Cys Gln Ser Tyr Thr Asp Ile Pro Trp Arg Glu Leu Phe Ala Pro
225                 230                 235                 240
```

9

```
AAG GGC GCC GAC GGC CGC ACG TTC GAG AAG TTC GTC GCG GAA TCG GGC    768
Lys Gly Ala Asp Gly Arg Thr Phe Glu Lys Phe Val Ala Glu Ser Gly
                245                 250                 255

GGC GCC GAG GCG ATC TGG TAC CCG TTC ACC GAG AAG CCG TGG ATG AAG    816
Gly Ala Glu Ala Ile Trp Tyr Pro Phe Thr Glu Lys Pro Trp Met Lys
            ---260                 265                 270

GTG TGG ACG GTC TCG CCG ACC AAG CCG GAC TCG TCG AAC GAG GTC GGA    864
Val Trp Thr Val Ser Pro Thr Lys Pro Asp Ser Ser Asn Glu Val Gly
            275                 280                 285

AGC CTC GGC TCG GCG GGC TCC CTC GTC GGC AAG CCT CCG CAG GCG CGT    912
Ser Leu Gly Ser Ala Gly Ser Leu Val Gly Lys Pro Pro Gln Ala Arg
    290                 295                 300

GAG GTC TCC GGC CCG TAC AAC TAC ATC TTC TCC GAC AAC CTG CCG GAG    960
Glu Val Ser Gly Pro Tyr Asn Tyr Ile Phe Ser Asp Asn Leu Pro Glu
305                 310                 315                 320

CCC ATC ACC GAC ATG ATC GGC GCC ATC AAC GCC GGA AAC CCC GGA ATC   1008
Pro Ile Thr Asp Met Ile Gly Ala Ile Asn Ala Gly Asn Pro Gly Ile
                325                 330                 335

GCA CCG CTG TTC GGC CCG GCG ATG TAC GAG ATC ACC AAG CTC GGG CTG   1056
Ala Pro Leu Phe Gly Pro Ala Met Tyr Glu Ile Thr Lys Leu Gly Leu
                340                 345                 350

GCC GCG ACG AAT GCC AAC GAC ATC TGG GGC TGG TCG AAG GAC GTC CAG   1104
Ala Ala Thr Asn Ala Asn Asp Ile Trp Gly Trp Ser Lys Asp Val Gln
                355                 360                 365

TTC TAC ATC AAG GCC ACG ACG TTG CGA CTC ACC GAG GGC GGC GGC GCC   1152
Phe Tyr Ile Lys Ala Thr Thr Leu Arg Leu Thr Glu Gly Gly Gly Ala
    370                 375                 380

GTC GTC ACG AGC CGC GCC AAC ATC GCG ACC GTG ATC AAC GAC TTC ACC   1200
Val Val Thr Ser Arg Ala Asn Ile Ala Thr Val Ile Asn Asp Phe Thr
385                 390                 395                 400

GAG TGG TTC CAC GAG CGC ATC GAG TTC TAC CGC GCG AAG GGC GAG TTC   1248
Glu Trp Phe His Glu Arg Ile Glu Phe Tyr Arg Ala Lys Gly Glu Phe
                405                 410                 415

CCG CTC AAC GGT CCG GTC GAG ATC CGC TGC TGC GGG CTC GAT CAG GCA   1296
Pro Leu Asn Gly Pro Val Glu Ile Arg Cys Cys Gly Leu Asp Gln Ala
                420                 425                 430

GCC GAC GTC AAG GTG CCG TCG GTG GGC CCG CCG ACC ATC TCG GCG ACC   1344
Ala Asp Val Lys Val Pro Ser Val Gly Pro Pro Thr Ile Ser Ala Thr
                435                 440                 445

CGT CCG CGT CCG GAT CAT CCG GAC TGG GAC GTC GCG ATC TGG CTG AAC   1392
Arg Pro Arg Pro Asp His Pro Asp Trp Asp Val Ala Ile Trp Leu Asn
    450                 455                 460

GTT CTC GGT GTT CCG GGC ACC CCC GGC ATG TTC GAG TTC TAC CGC GAG   1440
Val Leu Gly Val Pro Gly Thr Pro Gly Met Phe Glu Phe Tyr Arg Glu
465                 470                 475                 480

ATG GAG CAG TGG ATG CGG AGC CAC TAC AAC AAC GAC GAC GCC ACC TTC   1488
Met Glu Gln Trp Met Arg Ser His Tyr Asn Asn Asp Asp Ala Thr Phe
                485                 490                 495
```

```
CGG CCC GAG TGG TCG AAG GGG TGG GCG TTC GGT CCC GAC CCG TAC ACC        1536
Arg Pro Glu Trp Ser Lys Gly Trp Ala Phe Gly Pro Asp Pro Tyr Thr
            500             505             510

GAC AAC GAC ATC GTC ACG AAC AAG ATG CGC GCC ACC TAC ATC GAA GGT        1584
Asp Asn Asp Ile Val Thr Asn Lys Met Arg Ala Thr Tyr Ile Glu Gly
        515             520             525

GTC CCG ACG ACC GAG AAC TGG GAC ACC GCG CGC GCT CGG TAC AAC CAG        1632
Val Pro Thr Thr Glu Asn Trp Asp Thr Ala Arg Ala Arg Tyr Asn Gln
    530             535             540

ATC GAC CCG CAT CGC GTG TTC ACC AAC GGA TTC ATG GAC AAG CTG CTT        1680
Ile Asp Pro His Arg Val Phe Thr Asn Gly Phe Met Asp Lys Leu Leu
545             550             555             560

CCG                                                                    1683
Pro
```

(2) INFORMATION ZU SEQ ID NO: 2:

(i) SEQUENZ CHARAKTERISTIKA:

(A) LANGE: 561 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

```
Ser Thr Gly Pro Val Ala Pro Leu Pro Thr Pro Pro Asn Phe Pro Asn
 1               5                  10                  15

Asp Ile Ala Leu Phe Gln Gln Ala Tyr Gln Asn Trp Ser Lys Glu Ile
            20                  25                  30

Met Leu Asp Ala Thr Trp Val Cys Ser Pro Lys Thr Pro Gln Asp Val
        35                  40                  45

Val Arg Leu Ala Asn Trp Ala His Glu His Asp Tyr Lys Ile Arg Pro
    50                  55                  60

Arg Gly Ala Met His Gly Trp Thr Pro Leu Thr Val Glu Lys Gly Ala
65                  70                  75                      80

Asn Val Glu Lys Val Ile Leu Ala Asp Thr Met Thr His Leu Asn Gly
            85                  90                  95

Ile Thr Val Asn Thr Gly Gly Pro Val Ala Thr Val Thr Ala Gly Ala
            100                 105                 110

Gly Ala Ser Ile Glu Ala Ile Val Thr Glu Leu Gln Lys His Asp Leu
        115                 120                 125

Gly Trp Ala Asn Leu Pro Ala Pro Gly Val Leu Ser Ile Gly Gly Ala
    130                 135                 140

Leu Ala Val Asn Ala His Gly Ala Ala Leu Pro Ala Val Gly Gln Thr
145                 150                 155                 160

Thr Leu Pro Gly His Thr Tyr Gly Ser Leu Ser Asn Leu Val Thr Glu
            165                 170                 ·175

Leu Thr Ala Val Val Trp Asn Gly Thr Thr Tyr Ala Leu Glu Thr Tyr
            180                 185                 190

Gln Arg Asn Asp Pro Arg Ile Thr Pro Leu Leu Thr Asn Leu Gly Arg
    195                 200                 205

Cys Phe Leu Thr Ser Val Thr Met Gln Ala Gly Pro Asn Phe Arg Gln
    210                 215                 220

Arg Cys Gln Ser Tyr Thr Asp Ile Pro Trp Arg Glu Leu Phe Ala Pro
225                 230                 235                 240

Lys Gly Ala Asp Gly Arg Thr Phe Glu Lys Phe Val Ala Glu Ser Gly
            245                 250                 255

Gly Ala Glu Ala Ile Trp Tyr Pro Phe Thr Glu Lys Pro Trp Met Lys
            260                 265                 270
```

12

```
Val Trp Thr Val Ser Pro Thr Lys Pro Asp Ser Ser Asn Glu Val Gly
        275             280             285

Ser Leu Gly Ser Ala Gly Ser Leu Val Gly Lys Pro Pro Gln Ala Arg
        290             295             300

Glu Val Ser Gly Pro Tyr Asn Tyr Ile Phe Ser Asp Asn Leu Pro Glu
305             310             315             320

Pro Ile Thr Asp Met Ile Gly Ala Ile Asn Ala Gly Asn Pro Gly Ile
            325             330             335

Ala Pro Leu Phe Gly Pro Ala Met Tyr Glu Ile Thr Lys Leu Gly Leu
        340             345             350

Ala Ala Thr Asn Ala Asn Asp Ile Trp Gly Trp Ser Lys Asp Val Gln
        355             360             365

Phe Tyr Ile Lys Ala Thr Thr Leu Arg Leu Thr Glu Gly Gly Gly Ala
    370             375             380

Val Val Thr Ser Arg Ala Asn Ile Ala Thr Val Ile Asn Asp Phe Thr
385             390             395             400

Glu Trp Phe His Glu Arg Ile Glu Phe Tyr Arg Ala Lys Gly Glu Phe
            405             410             415

Pro Leu Asn Gly Pro Val Glu Ile Arg Cys Cys Gly Leu Asp Gln Ala
            420             425             430

Ala Asp Val Lys Val Pro Ser Val Gly Pro Pro Thr Ile Ser Ala Thr
        435             440             445

Arg Pro Arg Pro Asp His Pro Asp Trp Asp Val Ala Ile Trp Leu Asn
    450             455             460

Val Leu Gly Val Pro Gly Thr Pro Gly Met Phe Glu Phe Tyr Arg Glu
465             470             475             480

Met Glu Gln Trp Met Arg Ser His Tyr Asn Asn Asp Asp Ala Thr Phe
            485             490             495

Arg Pro Glu Trp Ser Lys Gly Trp Ala Phe Gly Pro Asp Pro Tyr Thr
        500             505             510

Asp Asn Asp Ile Val Thr Asn Lys Met Arg Ala Thr Tyr Ile Glu Gly
        515             520             525

Val Pro Thr Thr Glu Asn Trp Asp Thr Ala Arg Ala Arg Tyr Asn Gln
    530             535             540

Ile Asp Pro His Arg Val Phe Thr Asn Gly Phe Met Asp Lys Leu Leu
545             550             555             560

Pro
```

(i) SEQUENZ CHARAKTERISTIKA:

(A) LÄNGE: 48 Basenpaare
(B) ART: Nukleinsäure
(C) STRANGFORM: Einzel
(D) TOPOLOGIE: linear

(xi) SEQUENZBESCHREIBUNG: SEQ ID NO: 3:


TTCCCGCTCA ACGGTCCGGT CGAGATCCGC TGCTGCGGGC TCGATCAG       48


(2) INFORMATION ZU SEQ ID NO: 4:

(i) SEQUENZ CHARAKTERISTIKA:

(A) LÄNGE: 48 Basenpaare
(B) ART: Nukleinsäure
(C) STRANGFORM: Einzel
(D) TOPOLOGIE: linear

(xi) SEQUENZBESCHREIBUNG: SEQ ID NO: 4:


GCGATCTGGC TGAACGTTCT CGGTGTTCCG GGCACCCCCG GCATGTTC       48


(2) INFORMATION ZU SEQ ID NO: 5:

(i) SEQUENZ CHARAKTERISTIKA:

(A) LÄNGE: 36 Basenpaare
(B) ART: Nukleinsäure
(C) STRANGFORM: Einzel
(D) TOPOLOGIE: linear

(xi) SEQUENZBESCHREIBUNG: SEQ ID NO: 5:


GACGCCACCT TCCGGCCCGA GTGGTCGAAG GGGTGG       36


(2) INFORMATION ZU SEQ ID NO: 6:

(i) SEQUENZ CHARAKTERISTIKA:

(A) LANGE: 46 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 17..46

(xi) SEQUENZBESCHREIBUNG: SEQ ID NO: 6:

```
CACACAGGAA ACAGCT ATG ACC ATG ATT ACG CCA AGC TTG CAT GCC          46
                  Met Thr Met Ile Thr Pro Ser Leu His Ala
                   1               5                   10
```

(2) INFORMATION ZU SEQ ID NO: 7:

(i) SEQUENZ CHARAKTERISTIKA:

(A) LÄNGE: 10 Aminosäuren
(B) ART: Aminosäure
(D) TOPOLOGIE: linear

(ii) ART DES KOLEKULS: Protein
(xi) SEQUENZBESCHREIBUNG: SEQ ID NO: 7:

```
Met Thr Met Ile Thr Pro Ser Leu His Ala
 1               5                   10
```

(2) INFORMATION ZU SEQ ID NO: 8:

(i) SEQUENZ CHARAKTERISTIKA:

(A) LÄNGE: 49 Basenpaare
(B) ART: Nukleinsäure
(C) STRANGFORM: Einzel
(D) TOPOLOGIE: linear

(ix) MERKMALE:

(A) NAME/SCHLUSSEL: CDS
(B) LAGE: 20..49

(xi) SEQUENZBESCHREIBUNG: SEQ ID NO: 8:

```
GAATTTAAGG GGAACATCG ATG ACC ATG ATT ACG CCA AGC TTG CAT GCC          49
                     Met Thr Met Ile Thr Pro Ser Leu His Ala
                      1               5                   10
```

(2) INFORMATION ZU SEQ ID NO: 9:

(i) SEQUENZ CHARAKTERISTIKA:

(A) LÄNGE: 10 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 9:

```
Met Thr Met Ile Thr Pro Ser Leu His Ala
 1               5                   10
```

(2) INFORMATION ZU SEQ ID NO: 10:

(i) SEQUENZ CHARAKTERISTIKA:

(A) LÄNGE: 43 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 20..43

(xi) SEQUENZBESCHREIBUNG: SEQ ID NO: 10:

```
GAATTTAAGG GGAACATCG ATG AGT AAT CAC CAT GGG CAT GCC          43
                        Met Ser Asn His His Gly His Ala
                         1               5
```

.

(2) INFORMATION ZU SEQ ID NO: 11:

(i) SEQUENZ CHARAKTERISTIKA:

(A) LÄNGE: 8 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 11:

```
                        Met Ser Asn His His Gly His Ala
                         1               5
```

(2) INFORMATION ZU SEQ ID NO: 12:

(i) SEQUENZ CHARAKTERISTIKA:

(A) LÄNGE: 45 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 19..45

(xi) SEQUENZBESCHREIBUNG: SEQ ID NO: 12:

```
AATTTGGAGG GGAACATT ATG AGT AAT CAT CAC CAT GGG CAT GCC          45
                       Met Ser Asn His His His Gly His Ala
                        1               5
```

(2) INFORMATION ZU SEQ ID NO: 13:

   (i) SEQUENZ CHARAKTERISTIKA:

      (A) LÄNGE: 9 Aminosäuren
      (B) ART: Aminosäure
      (D) TOPOLOGIE: linear

   (ii) ART DES MOLEKÜLS: Protein
   (xi) SEQUENZBESCHREIBUNG: SEQ ID NO: 13:

```
Met Ser Asn His His His Gly His Ala
 1               5
```

(2) INFORMATION ZU SEQ ID NO: 14:

   (i) SEQUENZ CHARAKTERISTIKA:

      (A) LANGE: 58 Basenpaare
      (B) ART: Nukleinsäure
      (C) STRANGFORM: Einzel
      (D) TOPOLOGIE: linear

   (ix) MERKMALE:

      (A) NAME/SCHLÜSSEL: CDS
      (B) LAGE: 20..58

   (xi) SEQUENZBESCHREIBUNG: SEQ ID NO: 14:

```
GAATTTAAGG GGAACATCG ATG AGT AAT ACG CGT AAA CGC AAG CGC CGT ACG      52
                     Met Ser Asn Thr Arg Lys Arg Lys Arg Arg Thr
                      1               5                   10

CAT GCC                                                               58
His Ala
```

(2) INFORMATION ZU SEQ ID NO: 15:

   (i) SEQUENZ CHARAKTERISTIKA:

      (A) LÄNGE: 13 Aminosäuren
      (B) ART: Aminosäure
      (D) TOPOLOGIE: linear

   (ii) ART DES MOLEKÜLS: Protein
   (xi) SEQUENZBESCHREIBUNG: SEQ ID NO: 15:

```
Met Ser Asn Thr Arg Lys Arg Lys Arg Arg Thr His Ala
 1               5                   10
```

(2) INFORMATION ZU SEQ ID NO: 16:

   (i) SEQUENZ CHARAKTERISTIKA:

(A) LANGE: 48 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 25..48

(xi) SEQUENZBESCHREIBUNG: SEQ ID NO: 16:

```
GAATTCACAC AGGAAACAGA ATTC ATG GTT ATG CAC CAT GGG CAT GCC          48
                           Met Val Met His His Gly His Ala
                            1               5
```

(2) INFORMATION ZU SEQ ID NO: 17:

(i) SEQUENZ CHARAKTERISTIKA:

(A) LANGE: 8 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 17:

```
Met Val Met His His Gly His Ala
 1               5
```

(2) INFORMATION ZU SEQ ID NO: 18:

(i) SEQUENZ CHARAKTERISTIKA:

(A) LANGE: 1729 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 17..1729

(xi) SEQUENZBESCHREIBUNG: SEQ ID NO: 18:

```
CACACAGGAA ACAGCT ATG ACC ATG ATT ACG CCA AGC TTG CAT GCC TCG          49
                   Met Thr Met Ile Thr Pro Ser Leu His Ala Ser
                    1               5                  10


ACC GGG CCG GTC GCG CCG CTT CCG ACG CCG CCG AAC TTC CCG AAC GAC         97
Thr Gly Pro Val Ala Pro Leu Pro Thr Pro Pro Asn Phe Pro Asn Asp
            15              20                  25


ATC GCG CTG TTC CAG CAG GCG TAC CAG AAC TGG TCC AAG GAG ATC ATG        145
Ile Ala Leu Phe Gln Gln Ala Tyr Gln Asn Trp Ser Lys Glu Ile Met
            30              35                  40


CTG GAC GCC ACT TGG GTC TGC TCG CCC AAG ACG CCG CAG GAT GTC GTT        193
Leu Asp Ala Thr Trp Val Cys Ser Pro Lys Thr Pro Gln Asp Val Val
            45              50                  55


CGC CTT GCC AAC TGG GCG CAC GAG CAC GAC TAC AAG ATC CGC CCG CGC        241
Arg Leu Ala Asn Trp Ala His Glu His Asp Tyr Lys Ile Arg Pro Arg
     60              65              70                  75
```

EP 0 698 102 B1

```
GGC GCG ATG CAC GGC TGG ACC CCG CTC ACC GTG GAG AAG GGG GCC AAC          289
Gly Ala Met His Gly Trp Thr Pro Leu Thr Val Glu Lys Gly Ala Asn
                80                  85                  90

GTC GAG AAG GTG ATC CTC GCC GAC ACG ATG ACG CAT CTG AAC GGC ATC          337
Val Glu Lys Val Ile Leu Ala Asp Thr Met Thr His Leu Asn Gly Ile
                95                  100                 105

ACG GTG AAC ACG GGC GGC CCC GTG GCT ACC GTC ACC GCC GGT GCC GGC          385
Thr Val Asn Thr Gly Gly Pro Val Ala Thr Val Thr Ala Gly Ala Gly
                110                 115                 120

GCC AGC ATC GAG GCG ATC GTC ACC GAA CTG CAG AAG CAC GAC CTC GGC          433
Ala Ser Ile Glu Ala Ile Val Thr Glu Leu Gln Lys His Asp Leu Gly
        125                 130                 135

TGG GCC AAC CTG CCC GCT CCG GGT GTG CTG TCG ATC GGT GGC GCC CTT          481
Trp Ala Asn Leu Pro Ala Pro Gly Val Leu Ser Ile Gly Gly Ala Leu
140                 145                 150                 155

GCG GTC AAC GCG CAC GGT GCG GCG CTG CCG GCC GTC GGC CAG ACC ACG          529
Ala Val Asn Ala His Gly Ala Ala Leu Pro Ala Val Gly Gln Thr Thr
                160                 165                 170

CTG CCC GGT CAC ACC TAC GGT TCG CTG AGC AAC CTG GTC ACC GAG CTG          577
Leu Pro Gly His Thr Tyr Gly Ser Leu Ser Asn Leu Val Thr Glu Leu
                175                 180                 185

ACC GCG GTC GTC TGG AAC GGC ACC ACC TAC GCA CTC GAG ACG TAC CAG          625
Thr Ala Val Val Trp Asn Gly Thr Thr Tyr Ala Leu Glu Thr Tyr Gln
                190                 195                 200

CGC AAC GAT CCT CGG ATC ACC CCA CTG CTC ACC AAC CTC GGG CGC TGC          673
Arg Asn Asp Pro Arg Ile Thr Pro Leu Leu Thr Asn Leu Gly Arg Cys
        205                 210                 215

TTC CTG ACC TCG GTG ACG ATG CAG GCC GGC CCC AAC TTC CGT CAG CGG          721
Phe Leu Thr Ser Val Thr Met Gln Ala Gly Pro Asn Phe Arg Gln Arg
220                 225                 230                 235

TGC CAG AGC TAC ACC GAC ATC CCG TGG CGG GAA CTG TTC GCG CCG AAG          769
Cys Gln Ser Tyr Thr Asp Ile Pro Trp Arg Glu Leu Phe Ala Pro Lys
                240                 245                 250

GGC GCC GAC GGC CGC ACG TTC GAG AAG TTC GTC GCG GAA TCG GGC GGC          817
Gly Ala Asp Gly Arg Thr Phe Glu Lys Phe Val Ala Glu Ser Gly Gly
        255                 260                 265

GCC GAG GCG ATC TGG TAC CCG TTC ACC GAG AAG CCG TGG ATG AAG GTG          865
Ala Glu Ala Ile Trp Tyr Pro Phe Thr Glu Lys Pro Trp Met Lys Val
        270                 275                 280

TGG ACG GTC TCG CCG ACC AAG CCG GAC TCG TCG AAC GAG GTC GGA AGC          913
Trp Thr Val Ser Pro Thr Lys Pro Asp Ser Ser Asn Glu Val Gly Ser
        285                 290                 295

CTC GGC TCG GCG GGC TCC CTC GTC GGC AAG CCT CCG CAG GCG CGT GAG          961
Leu Gly Ser Ala Gly Ser Leu Val Gly Lys Pro Pro Gln Ala Arg Glu
300                 305                 310                 315

GTC TCC GGC CCG TAC AAC TAC ATC TTC TCC GAC AAC CTG CCG GAG CCC          1009
Val Ser Gly Pro Tyr Asn Tyr Ile Phe Ser Asp Asn Leu Pro Glu Pro
                320                 325                 330
```

20

```
ATC ACC GAC ATG ATC GGC GCC ATC AAC GCC GGA AAC CCC GGA ATC GCA     1057
Ile Thr Asp Met Ile Gly Ala Ile Asn Ala Gly Asn Pro Gly Ile Ala
            335             340             345

CCG CTG TTC GGC CCG GCG ATG TAC GAG ATC ACC AAG CTC GGG CTG GCC     1105
Pro Leu Phe Gly Pro Ala Met Tyr Glu Ile Thr Lys Leu Gly Leu Ala
            350             355             360

GCG ACG AAT GCC AAC GAC ATC TGG GGC TGG TCG AAG GAC GTC CAG TTC     1153
Ala Thr Asn Ala Asn Asp Ile Trp Gly Trp Ser Lys Asp Val Gln Phe
            365             370             375

TAC ATC AAG GCC ACG ACG TTG CGA CTC ACC GAG GGC GGC GGC GCC GTC     1201
Tyr Ile Lys Ala Thr Thr Leu Arg Leu Thr Glu Gly Gly Gly Ala Val
380             385             390             395

GTC ACG AGC CGC GCC AAC ATC GCG ACC GTG ATC AAC GAC TTC ACC GAG     1249
Val Thr Ser Arg Ala Asn Ile Ala Thr Val Ile Asn Asp Phe Thr Glu
            400             405             410

TGG TTC CAC GAG CGC ATC GAG TTC TAC CGC GCG AAG GGC GAG TTC CCG     1297
Trp Phe His Glu Arg Ile Glu Phe Tyr Arg Ala Lys Gly Glu Phe Pro
            415             420             425

CTC AAC GGT CCG GTC GAG ATC CGC TGC TGC GGG CTC GAT CAG GCA GCC     1345
Leu Asn Gly Pro Val Glu Ile Arg Cys Cys Gly Leu Asp Gln Ala Ala
            430             435             440

GAC GTC AAG GTG CCG TCG GTG GGC CCG CCG ACC ATC TCG GCG ACC CGT     1393
Asp Val Lys Val Pro Ser Val Gly Pro Pro Thr Ile Ser Ala Thr Arg
            445             450             455

CCG CGT CCG GAT CAT CCG GAC TGG GAC GTC GCG ATC TGG CTG AAC GTT     1441
Pro Arg Pro Asp His Pro Asp Trp Asp Val Ala Ile Trp Leu Asn Val
460             465             470             475

CTC GGT GTT CCG GGC ACC CCC GGC ATG TTC GAG TTC TAC CGC GAG ATG     1489
Leu Gly Val Pro Gly Thr Pro Gly Met Phe Glu Phe Tyr Arg Glu Met
            480             485             490

GAG CAG TGG ATG CGG AGC CAC TAC AAC AAC GAC GAC GCC ACC TTC CGG     1537
Glu Gln Trp Met Arg Ser His Tyr Asn Asn Asp Asp Ala Thr Phe Arg
            495             500             505

CCC GAG TGG TCG AAG GGG TGG GCG TTC GGT CCC GAC CCG TAC ACC GAC     1585
Pro Glu Trp Ser Lys Gly Trp Ala Phe Gly Pro Asp Pro Tyr Thr Asp
            510             515             520

AAC GAC ATC GTC ACG AAC AAG ATG CGC GCC ACC TAC ATC GAA GGT GTC     1633
Asn Asp Ile Val Thr Asn Lys Met Arg Ala Thr Tyr Ile Glu Gly Val
            525             530             535

CCG ACG ACC GAG AAC TGG GAC ACC GCG CGC GCT CGG TAC AAC CAG ATC     1681
Pro Thr Thr Glu Asn Trp Asp Thr Ala Arg Ala Arg Tyr Asn Gln Ile
540             545             550             555

GAC CCG CAT CGC GTG TTC ACC AAC GGA TTC ATG GAC AAG CTG CTT CCG     1729
Asp Pro His Arg Val Phe Thr Asn Gly Phe Met Asp Lys Leu Leu Pro
            560             565             570
```

(2) INFORMATION ZU SEQ ID NO: 19:

**EP 0 698 102 B1**

(i) SEQUENZ CHARAKTERISTIKA:

(A) LÄNGE: 571 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 19:

```
Met Thr Met Ile Thr Pro Ser Leu His Ala Ser Thr Gly Pro Val Ala
 1               5               10              15

Pro Leu Pro Thr Pro Pro Asn Phe Pro Asn Asp Ile Ala Leu Phe Gln
            20              25              30

Gln Ala Tyr Gln Asn Trp Ser Lys Glu Ile Met Leu Asp Ala Thr Trp
        35              40              45

Val Cys Ser Pro Lys Thr Pro Gln Asp Val Val Arg Leu Ala Asn Trp
        50              55              60

Ala His Glu His Asp Tyr Lys Ile Arg Pro Arg Gly Ala Met His Gly
65              70              75              80

Trp Thr Pro Leu Thr Val Glu Lys Gly Ala Asn Val Glu Lys Val Ile
            85              90              95

Leu Ala Asp Thr Met Thr His Leu Asn Gly Ile Thr Val Asn Thr Gly
            100             105             110

Gly Pro Val Ala Thr Val Thr Ala Gly Ala Gly Ala Ser Ile Glu Ala
        115             120             125

Ile Val Thr Glu Leu Gln Lys His Asp Leu Gly Trp Ala Asn Leu Pro
    130             135             140

Ala Pro Gly Val Leu Ser Ile Gly Gly Ala Leu Ala Val Asn Ala His
145             150             155             160

Gly Ala Ala Leu Pro Ala Val Gly Gln Thr Thr Leu Pro Gly His Thr
            165             170             175

Tyr Gly Ser Leu Ser Asn Leu Val Thr Glu Leu Thr Ala Val Val Trp
            180             185             190

Asn Gly Thr Thr Tyr Ala Leu Glu Thr Tyr Gln Arg Asn Asp Pro Arg
            195             200             205

Ile Thr Pro Leu Leu Thr Asn Leu Gly Arg Cys Phe Leu Thr Ser Val
    210             215             220

Thr Met Gln Ala Gly Pro Asn Phe Arg Gln Arg Cys Gln Ser Tyr Thr
225             230             235             240

Asp Ile Pro Trp Arg Glu Leu Phe Ala Pro Lys Gly Ala Asp Gly Arg
            245             250             255

Thr Phe Glu Lys Phe Val Ala Glu Ser Gly Gly Ala Glu Ala Ile Trp
            260             265             270
```

22

```
Tyr Pro Phe Thr Glu Lys Pro Trp Met Lys Val Trp Thr Val Ser Pro
        275             280             285

Thr Lys Pro Asp Ser Ser Asn Glu Val Gly Ser Leu Gly Ser Ala Gly
        290             295             300

Ser Leu Val Gly Lys Pro Pro Gln Ala Arg Glu Val Ser Gly Pro Tyr
305             310             315             320

Asn Tyr Ile Phe Ser Asp Asn Leu Pro Glu Pro Ile Thr Asp Met Ile
                325             330             335

Gly Ala Ile Asn Ala Gly Asn Pro Gly Ile Ala Pro Leu Phe Gly Pro
            340             345             350

Ala Met Tyr Glu Ile Thr Lys Leu Gly Leu Ala Ala Thr Asn Ala Asn
        355             360             365

Asp Ile Trp Gly Trp Ser Lys Asp Val Gln Phe Tyr Ile Lys Ala Thr
    370             375             380

Thr Leu Arg Leu Thr Glu Gly Gly Gly Ala Val Val Thr Ser Arg Ala
385             390             395             400

Asn Ile Ala Thr Val Ile Asn Asp Phe Thr Glu Trp Phe His Glu Arg
            405             410             415

Ile Glu Phe Tyr Arg Ala Lys Gly Glu Phe Pro Leu Asn Gly Pro Val
        420             425             430

Glu Ile Arg Cys Cys Gly Leu Asp Gln Ala Ala Asp Val Lys Val Pro
        435             440             445

Ser Val Gly Pro Pro Thr Ile Ser Ala Thr Arg Pro Arg Pro Asp His
    450             455             460

Pro Asp Trp Asp Val Ala Ile Trp Leu Asn Val Leu Gly Val Pro Gly
465             470             475             480

Thr Pro Gly Met Phe Glu Phe Tyr Arg Glu Met Glu Gln Trp Met Arg
            485             490             495

Ser His Tyr Asn Asn Asp Asp Ala Thr Phe Arg Pro Glu Trp Ser Lys
        500             505             510

Gly Trp Ala Phe Gly Pro Asp Pro Tyr Thr Asp Asn Asp Ile Val Thr
        515             520             525

Asn Lys Met Arg Ala Thr Tyr Ile Glu Gly Val Pro Thr Thr Glu Asn
    530             535             540

Trp Asp Thr Ala Arg Ala Arg Tyr Asn Gln Ile Asp Pro His Arg Val
545             550             555             560

Phe Thr Asn Gly Phe Met Asp Lys Leu Leu Pro
            565             570
```

(2) INFORMATION ZU SEQ ID NO: 20:

(i) SEQUENZ CHARAKTERISTIKA:

(A) LÄNGE: 1732 Basenpaare

(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 20..1732

(xi) SEQUENZBESCHREIBUNG: SEQ ID NO: 20:

```
GAATTTAAGG GGAACATCG ATG ACC ATG ATT ACG CCA AGC TTG CAT GCC TCG      52
                     Met Thr Met Ile Thr Pro Ser Leu His Ala Ser
                      1           5                   10

ACC GGG CCG GTC GCG CCG CTT CCG ACG CCG CCG AAC TTC CCG AAC GAC      100
Thr Gly Pro Val Ala Pro Leu Pro Thr Pro Pro Asn Phe Pro Asn Asp
            15              20              25

ATC GCG CTG TTC CAG CAG GCG TAC CAG AAC TGG TCC AAG GAG ATC ATG      148
Ile Ala Leu Phe Gln Gln Ala Tyr Gln Asn Trp Ser Lys Glu Ile Met
            30              35              40

CTG GAC GCC ACT TGG GTC TGC TCG CCC AAG ACG CCG CAG GAT GTC GTT      196
Leu Asp Ala Thr Trp Val Cys Ser Pro Lys Thr Pro Gln Asp Val Val
            45              50                  55

CGC CTT GCC AAC TGG GCG CAC GAG CAC GAC TAC AAG ATC CGC CCG CGC      244
Arg Leu Ala Asn Trp Ala His Glu His Asp Tyr Lys Ile Arg Pro Arg
 60              65              70                  75

GGC GCG ATG CAC GGC TGG ACC CCG CTC ACC GTG GAG AAG GGG GCC AAC      292
Gly Ala Met His Gly Trp Thr Pro Leu Thr Val Glu Lys Gly Ala Asn
                80              85              90

GTC GAG AAG GTG ATC CTC GCC GAC ACG ATG ACG CAT CTG AAC GGC ATC      340
Val Glu Lys Val Ile Leu Ala Asp Thr Met Thr His Leu Asn Gly Ile
            95              100             105

ACG GTG AAC ACG GGC GGC CCC GTG GCT ACC GTC ACC GCC GGT GCC GGC      388
Thr Val Asn Thr Gly Gly Pro Val Ala Thr Val Thr Ala Gly Ala Gly
            110             115             120

GCC AGC ATC GAG GCG ATC GTC ACC GAA CTG CAG AAG CAC GAC CTC GGC      436
Ala Ser Ile Glu Ala Ile Val Thr Glu Leu Gln Lys His Asp Leu Gly
            125             130             135

TGG GCC AAC CTG CCC GCT CCG GGT GTG CTG TCG ATC GGT GGC GCC CTT      484
Trp Ala Asn Leu Pro Ala Pro Gly Val Leu Ser Ile Gly Gly Ala Leu
140             145             150             155

GCG GTC AAC GCG CAC GGT GCG GCG CTG CCG GCC GTC GGC CAG ACC ACG      532
Ala Val Asn Ala His Gly Ala Ala Leu Pro Ala Val Gly Gln Thr Thr
            160             165             170

CTG CCC GGT CAC ACC TAC GGT TCG CTG AGC AAC CTG GTC ACC GAG CTG      580
Leu Pro Gly His Thr Tyr Gly Ser Leu Ser Asn Leu Val Thr Glu Leu
            175             180             185
```

```
ACC GCG GTC GTC TGG AAC GGC ACC ACC TAC GCA CTC GAG ACG TAC CAG        628
Thr Ala Val Val Trp Asn Gly Thr Thr Tyr Ala Leu Glu Thr Tyr Gln
        190             195             200

CGC AAC GAT CCT CGG ATC ACC CCA CTG CTC ACC AAC CTC GGG CGC TGC        676
Arg Asn Asp Pro Arg Ile Thr Pro Leu Leu Thr Asn Leu Gly Arg Cys
    205             210             215

TTC CTG ACC TCG GTG ACG ATG CAG GCC GGC CCC AAC TTC CGT CAG CGG        724
Phe Leu Thr Ser Val Thr Met Gln Ala Gly Pro Asn Phe Arg Gln Arg
220             225             230             235

TGC CAG AGC TAC ACC GAC ATC CCG TGG CGG GAA CTG TTC GCG CCG AAG        772
Cys Gln Ser Tyr Thr Asp Ile Pro Trp Arg Glu Leu Phe Ala Pro Lys
            240             245             250

GGC GCC GAC GGC CGC ACG TTC GAG AAG TTC GTC GCG GAA TCG GGC GGC        820
Gly Ala Asp Gly Arg Thr Phe Glu Lys Phe Val Ala Glu Ser Gly Gly
            255             260             265

GCC GAG GCG ATC TGG TAC CCG TTC ACC GAG AAG CCG TGG ATG AAG GTG        868
Ala Glu Ala Ile Trp Tyr Pro Phe Thr Glu Lys Pro Trp Met Lys Val
            270             275             280

TGG ACG GTC TCG CCG ACC AAG CCG GAC TCG TCG AAC GAG GTC GGA AGC        916
Trp Thr Val Ser Pro Thr Lys Pro Asp Ser Ser Asn Glu Val Gly Ser
        285             290             295

CTC GGC TCG GCG GGC TCC CTC GTC GGC AAG CCT CCG CAG GCG CGT GAG        964
Leu Gly Ser Ala Gly Ser Leu Val Gly Lys Pro Pro Gln Ala Arg Glu
300             305             310             315

GTC TCC GGC CCG TAC AAC TAC ATC TTC TCC GAC AAC CTG CCG GAG CCC        1012
Val Ser Gly Pro Tyr Asn Tyr Ile Phe Ser Asp Asn Leu Pro Glu Pro
            320             325             330

ATC ACC GAC ATG ATC GGC GCC ATC AAC GCC GGA AAC CCC GGA ATC GCA        1060
Ile Thr Asp Met Ile Gly Ala Ile Asn Ala Gly Asn Pro Gly Ile Ala
            335             340             345

CCG CTG TTC GGC CCG GCG ATG TAC GAG ATC ACC AAG CTC GGG CTG GCC        1108
Pro Leu Phe Gly Pro Ala Met Tyr Glu Ile Thr Lys Leu Gly Leu Ala
            350             355             360

GCG ACG AAT GCC AAC GAC ATC TGG GGC TGG TCG AAG GAC GTC CAG TTC        1156
Ala Thr Asn Ala Asn Asp Ile Trp Gly Trp Ser Lys Asp Val Gln Phe
        365             370             375

TAC ATC AAG GCC ACG ACG TTG CGA CTC ACC GAG GGC GGC GGC GCC GTC        1204
Tyr Ile Lys Ala Thr Thr Leu Arg Leu Thr Glu Gly Gly Gly Ala Val
380             385             390             395

GTC ACG AGC CGC GCC AAC ATC GCG ACC GTG ATC AAC GAC TTC ACC GAG        1252
Val Thr Ser Arg Ala Asn Ile Ala Thr Val Ile Asn Asp Phe Thr Glu
            400             405             410

TGG TTC CAC GAG CGC ATC GAG TTC TAC CGC GCG AAG GGC GAG TTC CCG        1300
Trp Phe His Glu Arg Ile Glu Phe Tyr Arg Ala Lys Gly Glu Phe Pro
            415             420             425

CTC AAC GGT CCG GTC GAG ATC CGC TGC TGC GGG CTC GAT CAG GCA GCC        1348
Leu Asn Gly Pro Val Glu Ile Arg Cys Cys Gly Leu Asp Gln Ala Ala
            430             435             440
```

```
GAC GTC AAG GTG CCG TCG GTG GGC CCG CCG ACC ATC TCG GCG ACC CGT      1396
Asp Val Lys Val Pro Ser Val Gly Pro Pro Thr Ile Ser Ala Thr Arg
        445                 450                 455

CCG CGT CCG GAT CAT CCG GAC TGG GAC GTC GCG ATC TGG CTG AAC GTT      1444
Pro Arg Pro Asp His Pro Asp Trp Asp Val Ala Ile Trp Leu Asn Val
460                     465                 470                 475

CTC GGT GTT CCG GGC ACC CCC GGC ATG TTC GAG TTC TAC CGC GAG ATG      1492
Leu Gly Val Pro Gly Thr Pro Gly Met Phe Glu Phe Tyr Arg Glu Met
                480                 485                 490

GAG CAG TGG ATG CGG AGC CAC TAC AAC AAC GAC GAC GCC ACC TTC CGG      1540
Glu Gln Trp Met Arg Ser His Tyr Asn Asn Asp Asp Ala Thr Phe Arg
                495                 500                 505

CCC GAG TGG TCG AAG GGG TGG GCG TTC GGT CCC GAC CCG TAC ACC GAC      1588
Pro Glu Trp Ser Lys Gly Trp Ala Phe Gly Pro Asp Pro Tyr Thr Asp
                510                 515                 520

AAC GAC ATC GTC ACG AAC AAG ATG CGC GCC ACC TAC ATC GAA GGT GTC      1636
Asn Asp Ile Val Thr Asn Lys Met Arg Ala Thr Tyr Ile Glu Gly Val
                525                 530                 535

CCG ACG ACC GAG AAC TGG GAC ACC GCG CGC GCT CGG TAC AAC CAG ATC      1684
Pro Thr Thr Glu Asn Trp Asp Thr Ala Arg Ala Arg Tyr Asn Gln Ile
540                     545                 550                 555

GAC CCG CAT CGC GTG TTC ACC AAC GGA TTC ATG GAC AAG CTG CTT CCG      1732
Asp Pro His Arg Val Phe Thr Asn Gly Phe Met Asp Lys Leu Leu Pro
                560                 565                 570
```

(2) INFORMATION ZU SEQ ID NO: 21:

    (i) SEQUENZ CHARAKTERISTIKA:

        (A) LANGE: 571 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 21:

```
Met Thr Met Ile Thr Pro Ser Leu His Ala Ser Thr Gly Pro Val Ala
 1               5               10              15

Pro Leu Pro Thr Pro Pro Asn Phe Pro Asn Asp Ile Ala Leu Phe Gln
            20              25              30

Gln Ala Tyr Gln Asn Trp Ser Lys Glu Ile Met Leu Asp Ala Thr Trp
        35              40              45

Val Cys Ser Pro Lys Thr Pro Gln Asp Val Val Arg Leu Ala Asn Trp
    50              55              60

Ala His Glu His Asp Tyr Lys Ile Arg Pro Arg Gly Ala Met His Gly
65              70              75              80

Trp Thr Pro Leu Thr Val Glu Lys Gly Ala Asn Val Glu Lys Val Ile
            85              90              95
```

EP 0 698 102 B1

```
Leu Ala Asp Thr Met Thr His Leu Asn Gly Ile Thr Val Asn Thr Gly
        100                 105             110

Gly Pro Val Ala Thr Val Thr Ala Gly Ala Gly Ala Ser Ile Glu Ala
        115                 120             125

Ile Val Thr Glu Leu Gln Lys His Asp Leu Gly Trp Ala Asn Leu Pro
    130                 135             140

Ala Pro Gly Val Leu Ser Ile Gly Gly Ala Leu Ala Val Asn Ala His
145             150                 155             160

Gly Ala Ala Leu Pro Ala Val Gly Gln Thr Thr Leu Pro Gly His Thr
            165                 170             175

Tyr Gly Ser Leu Ser Asn Leu Val Thr Glu Leu Thr Ala Val Val Trp
            180                 185             190

Asn Gly Thr Thr Tyr Ala Leu Glu Thr Tyr Gln Arg Asn Asp Pro Arg
        195                 200             205

Ile Thr Pro Leu Leu Thr Asn Leu Gly Arg Cys Phe Leu Thr Ser Val
    210                 215             220

Thr Met Gln Ala Gly Pro Asn Phe Arg Gln Arg Cys Gln Ser Tyr Thr
225             230                 235             240

Asp Ile Pro Trp Arg Glu Leu Phe Ala Pro Lys Gly Ala Asp Gly Arg
            245                 250             255

Thr Phe Glu Lys Phe Val Ala Glu Ser Gly Gly Ala Glu Ala Ile Trp
            260                 265             270

Tyr Pro Phe Thr Glu Lys Pro Trp Met Lys Val Trp Thr Val Ser Pro
            275                 280             285

Thr Lys Pro Asp Ser Ser Asn Glu Val Gly Ser Leu Gly Ser Ala Gly
        290                 295             300

Ser Leu Val Gly Lys Pro Pro Gln Ala Arg Glu Val Ser Gly Pro Tyr
305             310                 315             320

Asn Tyr Ile Phe Ser Asp Asn Leu Pro Glu Pro Ile Thr Asp Met Ile
            325                 330             335

Gly Ala Ile Asn Ala Gly Asn Pro Gly Ile Ala Pro Leu Phe Gly Pro
            340                 345             350

Ala Met Tyr Glu Ile Thr Lys Leu Gly Leu Ala Ala Thr Asn Ala Asn
        355                 360             365

Asp Ile Trp Gly Trp Ser Lys Asp Val Gln Phe Tyr Ile Lys Ala Thr
    370                 375             380

Thr Leu Arg Leu Thr Glu Gly Gly Gly Ala Val Val Thr Ser Arg Ala
385             390                 395             400

Asn Ile Ala Thr Val Ile Asn Asp Phe Thr Glu Trp Phe His Glu Arg
            405                 410             415

Ile Glu Phe Tyr Arg Ala Lys Gly Glu Phe Pro Leu Asn Gly Pro Val
            420                 425             430
```

28

```
Glu Ile Arg Cys Cys Gly Leu Asp Gln Ala Ala Asp Val Lys Val Pro
    435                 440             445

Ser Val Gly Pro Pro Thr Ile Ser Ala Thr Arg Pro Arg Pro Asp His
    450                 455             460

Pro Asp Trp Asp Val Ala Ile Trp Leu Asn Val Leu Gly Val Pro Gly
465                 470                 475                 480

Thr Pro Gly Met Phe Glu Phe Tyr Arg Glu Met Glu Gln Trp Met Arg
                485                 490                 495

Ser His Tyr Asn Asn Asp Asp Ala Thr Phe Arg Pro Glu Trp Ser Lys
                500                 505             510

Gly Trp Ala Phe Gly Pro Asp Pro Tyr Thr Asp Asn Asp Ile Val Thr
    515                 520                 525

Asn Lys Met Arg Ala Thr Tyr Ile Glu Gly Val Pro Thr Thr Glu Asn
    530                 535                 540

Trp Asp Thr Ala Arg Ala Arg Tyr Asn Gln Ile Asp Pro His Arg Val
545                 550                 555                 560

Phe Thr Asn Gly Phe Met Asp Lys Leu Leu Pro
                565                 570
```

(2) INFORMATION ZU SEQ ID NO: 22:

  (i) SEQUENZ CHARAKTERISTIKA:

    (A) LÄNGE: 1726 Basenpaare
    (B) ART: Nukleinsäure
    (C) STRANGFORM: Einzel
    (D) TOPOLOGIE: linear

  (ix) MERKMALE:

    (A) NAME/SCHLÜSSEL: CDS
    (B) LAGE: 20..1726

  (xi) SEQUENZ BESCHREIBUNG: SEQ ID NO: 22:

```
GAATTTAAGG GGAACATCG ATG AGT AAT CAC CAT GGG CAT GCC TCG ACC GGG          52
                         Met Ser Asn His His Gly His Ala Ser Thr Gly
                          1              5                  10

CCG GTC GCG CCG CTT CCG ACG CCG CCG AAC TTC CCG AAC GAC ATC GCG          100
Pro Val Ala Pro Leu Pro Thr Pro Pro Asn Phe Pro Asn Asp Ile Ala
            15                  20                  25

CTG TTC CAG CAG GCG TAC CAG AAC TGG TCC AAG GAG ATC ATG CTG GAC          148
Leu Phe Gln Gln Ala Tyr Gln Asn Trp Ser Lys Glu Ile Met Leu Asp
        30                  35                  40

GCC ACT TGG GTC TGC TCG CCC AAG ACG CCG CAG GAT GTC GTT CGC CTT          196
Ala Thr Trp Val Cys Ser Pro Lys Thr Pro Gln Asp Val Val Arg Leu
        45                  50                  55

GCC AAC TGG GCG CAC GAG CAC GAC TAC AAG ATC CGC CCG CGC GGC GCG          244
Ala Asn Trp Ala His Glu His Asp Tyr Lys Ile Arg Pro Arg Gly Ala
 60             65                  70                  75
```

```
ATG CAC GGC TGG ACC CCG CTC ACC GTG GAG AAG GGG GCC AAC GTC GAG         292
Met His Gly Trp Thr Pro Leu Thr Val Glu Lys Gly Ala Asn Val Glu
                80                      85                  90

AAG GTG ATC CTC GCC GAC ACG ATG ACG CAT CTG AAC GGC ATC ACG GTG         340
Lys Val Ile Leu Ala Asp Thr Met Thr His Leu Asn Gly Ile Thr Val
                95                     100                 105

AAC ACG GGC GGC CCC GTG GCT ACC GTC ACC GCC GGT GCC GGC GCC AGC         388
Asn Thr Gly Gly Pro Val Ala Thr Val Thr Ala Gly Ala Gly Ala Ser
            110                     115                 120

ATC GAG GCG ATC GTC ACC GAA CTG CAG AAG CAC GAC CTC GGC TGG GCC         436
Ile Glu Ala Ile Val Thr Glu Leu Gln Lys His Asp Leu Gly Trp Ala
            125                     130             135

AAC CTG CCC GCT CCG GGT GTG CTG TCG ATC GGT GGC GCC CTT GCG GTC         484
Asn Leu Pro Ala Pro Gly Val Leu Ser Ile Gly Gly Ala Leu Ala Val
140             145                     150                 155

AAC GCG CAC GGT GCG GCG CTG CCG GCC GTC GGC CAG ACC ACG CTG CCC         532
Asn Ala His Gly Ala Ala Leu Pro Ala Val Gly Gln Thr Thr Leu Pro
            160                     165                 170

GGT CAC ACC TAC GGT TCG CTG AGC AAC CTG GTC ACC GAG CTG ACC GCG         580
Gly His Thr Tyr Gly Ser Leu Ser Asn Leu Val Thr Glu Leu Thr Ala
                175                     180             185

GTC GTC TGG AAC GGC ACC ACC TAC GCA CTC GAG ACG TAC CAG CGC AAC         628
Val Val Trp Asn Gly Thr Thr Tyr Ala Leu Glu Thr Tyr Gln Arg Asn
            190                     195                 200

GAT CCT CGG ATC ACC CCA CTG CTC ACC AAC CTC GGG CGC TGC TTC CTG         676
Asp Pro Arg Ile Thr Pro Leu Leu Thr Asn Leu Gly Arg Cys Phe Leu
        205                     210                 215

ACC TCG GTG ACG ATG CAG GCC GGC CCC AAC TTC CGT CAG CGG TGC CAG         724
Thr Ser Val Thr Met Gln Ala Gly Pro Asn Phe Arg Gln Arg Cys Gln
220                     225                     230                 235

AGC TAC ACC GAC ATC CCG TGG CGG GAA CTG TTC GCG CCG AAG GGC GCC         772
Ser Tyr Thr Asp Ile Pro Trp Arg Glu Leu Phe Ala Pro Lys Gly Ala
            240                     245                 250

GAC GGC CGC ACG TTC GAG AAG TTC GTC GCG GAA TCG GGC GGC GCC GAG         820
Asp Gly Arg Thr Phe Glu Lys Phe Val Ala Glu Ser Gly Gly Ala Glu
            255                     260             265

GCG ATC TGG TAC CCG TTC ACC GAG AAG CCG TGG ATG AAG GTG TGG ACG         868
Ala Ile Trp Tyr Pro Phe Thr Glu Lys Pro Trp Met Lys Val Trp Thr
            270                     275             280

GTC TCG CCG ACC AAG CCG GAC TCG TCG AAC GAG GTC GGA AGC CTC GGC         916
Val Ser Pro Thr Lys Pro Asp Ser Ser Asn Glu Val Gly Ser Leu Gly
            285                     290             295

TCG GCG GGC TCC CTC GTC GGC AAG CCT CCG CAG GCG CGT GAG GTC TCC         964
Ser Ala Gly Ser Leu Val Gly Lys Pro Pro Gln Ala Arg Glu Val Ser
300                     305                 310                 315

GGC CCG TAC AAC TAC ATC TTC TCC GAC AAC CTG CCG GAG CCC ATC ACC         1012
Gly Pro Tyr Asn Tyr Ile Phe Ser Asp Asn Leu Pro Glu Pro Ile Thr
                320                     325                 330
```

```
GAC ATG ATC GGC GCC ATC AAC GCC GGA AAC CCC GGA ATC GCA CCG CTG        1060
Asp Met Ile Gly Ala Ile Asn Ala Gly Asn Pro Gly Ile Ala Pro Leu
        335             340             345

TTC GGC CCG GCG ATG TAC GAG ATC ACC AAG CTC GGG CTG GCC GCG ACG        1108
Phe Gly Pro Ala Met Tyr Glu Ile Thr Lys Leu Gly Leu Ala Ala Thr
        350             355             360

AAT GCC AAC GAC ATC TGG GGC TGG TCG AAG GAC GTC CAG TTC TAC ATC        1156
Asn Ala Asn Asp Ile Trp Gly Trp Ser Lys Asp Val Gln Phe Tyr Ile
        365             370             375

AAG GCC ACG ACG TTG CGA CTC ACC GAG GGC GGC GGC GCC GTC GTC ACG        1204
Lys Ala Thr Thr Leu Arg Leu Thr Glu Gly Gly Gly Ala Val Val Thr
        380             385             390             395

AGC CGC GCC AAC ATC GCG ACC GTG ATC AAC GAC TTC ACC GAG TGG TTC        1252
Ser Arg Ala Asn Ile Ala Thr Val Ile Asn Asp Phe Thr Glu Trp Phe
                400             405             410

CAC GAG CGC ATC GAG TTC TAC CGC GCG AAG GGC GAG TTC CCG CTC AAC        1300
His Glu Arg Ile Glu Phe Tyr Arg Ala Lys Gly Glu Phe Pro Leu Asn
                415             420             425

GGT CCG GTC GAG ATC CGC TGC TGC GGG CTC GAT CAG GCA GCC GAC GTC        1348
Gly Pro Val Glu Ile Arg Cys Cys Gly Leu Asp Gln Ala Ala Asp Val
                430             435             440

AAG GTG CCG TCG GTG GGC CCG CCG ACC ATC TCG GCG ACC CGT CCG CGT        1396
Lys Val Pro Ser Val Gly Pro Pro Thr Ile Ser Ala Thr Arg Pro Arg
        445             450             455

CCG GAT CAT CCG GAC TGG GAC GTC GCG ATC TGG CTG AAC GTT CTC GGT        1444
Pro Asp His Pro Asp Trp Asp Val Ala Ile Trp Leu Asn Val Leu Gly
460             465             470             475

GTT CCG GGC ACC CCC GGC ATG TTC GAG TTC TAC CGC GAG ATG GAG CAG        1492
Val Pro Gly Thr Pro Gly Met Phe Glu Phe Tyr Arg Glu Met Glu Gln
                480             485             490

TGG ATG CGG AGC CAC TAC AAC AAC GAC GAC GCC ACC TTC CGG CCC GAG        1540
Trp Met Arg Ser His Tyr Asn Asn Asp Asp Ala Thr Phe Arg Pro Glu
                495             500             505

TGG TCG AAG GGG TGG GCG TTC GGT CCC GAC CCG TAC ACC GAC AAC GAC        1588
Trp Ser Lys Gly Trp Ala Phe Gly Pro Asp Pro Tyr Thr Asp Asn Asp
        510             515             520

ATC GTC ACG AAC AAG ATG CGC GCC ACC TAC ATC GAA GGT GTC CCG ACG        1636
Ile Val Thr Asn Lys Met Arg Ala Thr Tyr Ile Glu Gly Val Pro Thr
        525             530             535

ACC GAG AAC TGG GAC ACC GCG CGC GCT CGG TAC AAC CAG ATC GAC CCG        1684
Thr Glu Asn Trp Asp Thr Ala Arg Ala Arg Tyr Asn Gln Ile Asp Pro
540             545             550             555

CAT CGC GTG TTC ACC AAC GGA TTC ATG GAC AAG CTG CTT CCG                1726
His Arg Val Phe Thr Asn Gly Phe Met Asp Lys Leu Leu Pro
                560             565
```

(2) INFORMATION ZU SEQ ID NO: 23:

(i) SEQUENZ CHARAKTERISTIKA:

(A) LANGE: 569 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 23:

```
Met Ser Asn His His Gly His Ala Ser Thr Gly Pro Val Ala Pro Leu
1               5                   10              15

Pro Thr Pro Pro Asn Phe Pro Asn Asp Ile Ala Leu Phe Gln Gln Ala
        20              25                  30

Tyr Gln Asn Trp Ser Lys Glu Ile Met Leu Asp Ala Thr Trp Val Cys
        35              40                  45

Ser Pro Lys Thr Pro Gln Asp Val Val Arg Leu Ala Asn Trp Ala His
    50              55              60

Glu His Asp Tyr Lys Ile Arg Pro Arg Gly Ala Met His Gly Trp Thr
65              70              75                  80

Pro Leu Thr Val Glu Lys Gly Ala Asn Val Glu Lys Val Ile Leu Ala
            85              90              95

Asp Thr Met Thr His Leu Asn Gly Ile Thr Val Asn Thr Gly Gly Pro
        100             105             110

Val Ala Thr Val Thr Ala Gly Ala Gly Ala Ser Ile Glu Ala Ile Val
        115             120             125

Thr Glu Leu Gln Lys His Asp Leu Gly Trp Ala Asn Leu Pro Ala Pro
    130             135             140

Gly Val Leu Ser Ile Gly Gly Ala Leu Ala Val Asn Ala His Gly Ala
145             150             155             160

Ala Leu Pro Ala Val Gly Gln Thr Thr Leu Pro Gly His Thr Tyr Gly
            165             170             175

Ser Leu Ser Asn Leu Val Thr Glu Leu Thr Ala Val Val Trp Asn Gly
            180             185             190

Thr Thr Tyr Ala Leu Glu Thr Tyr Gln Arg Asn Asp Pro Arg Ile Thr
        195             200             205

Pro Leu Leu Thr Asn Leu Gly Arg Cys Phe Leu Thr Ser Val Thr Met
    210             215             220

Gln Ala Gly Pro Asn Phe Arg Gln Arg Cys Gln Ser Tyr Thr Asp Ile
225             230             235             240

Pro Trp Arg Glu Leu Phe Ala Pro Lys Gly Ala Asp Gly Arg Thr Phe
            245             250             255

Glu Lys Phe Val Ala Glu Ser Gly Gly Ala Glu Ala Ile Trp Tyr Pro
        260             265             270
```

```
        Phe Thr Glu Lys Pro Trp Met Lys Val Trp Thr Val Ser Pro Thr Lys
                275             280             285

        Pro Asp Ser Ser Asn Glu Val Gly Ser Leu Gly Ser Ala Gly Ser Leu
                290             295             300

        Val Gly Lys Pro Pro Gln Ala Arg Glu Val Ser Gly Pro Tyr Asn Tyr
        305             310             315             320

        Ile Phe Ser Asp Asn Leu Pro Glu Pro Ile Thr Asp Met Ile Gly Ala
                325             330             335

        Ile Asn Ala Gly Asn Pro Gly Ile Ala Pro Leu Phe Gly Pro Ala Met
                    340             345             350

        Tyr Glu Ile Thr Lys Leu Gly Leu Ala Ala Thr Asn Ala Asn Asp Ile
                355             360             365

        Trp Gly Trp Ser Lys Asp Val Gln Phe Tyr Ile Lys Ala Thr Thr Leu
            370             375             380

        Arg Leu Thr Glu Gly Gly Gly Ala Val Val Thr Ser Arg Ala Asn Ile
        385             390             395             400

        Ala Thr Val Ile Asn Asp Phe Thr Glu Trp Phe His Glu Arg Ile Glu
                    405             410                 415

        Phe Tyr Arg Ala Lys Gly Glu Phe Pro Leu Asn Gly Pro Val Glu Ile
                420             425             430

        Arg Cys Cys Gly Leu Asp Gln Ala Ala Asp Val Lys Val Pro Ser Val
                435             440             445

        Gly Pro Pro Thr Ile Ser Ala Thr Arg Pro Arg Pro Asp His Pro Asp
            450             455             460

        Trp Asp Val Ala Ile Trp Leu Asn Val Leu Gly Val Pro Gly Thr Pro
        465             470             475             480

        Gly Met Phe Glu Phe Tyr Arg Glu Met Glu Gln Trp Met Arg Ser His
                    485             490             495

        Tyr Asn Asn Asp Asp Ala Thr Phe Arg Pro Glu Trp Ser Lys Gly Trp
                500             505             510

        Ala Phe Gly Pro Asp Pro Tyr Thr Asp Asn Asp Ile Val Thr Asn Lys
                515             520             525

        Met Arg Ala Thr Tyr Ile Glu Gly Val Pro Thr Thr Glu Asn Trp Asp
            530             535             540

        Thr Ala Arg Ala Arg Tyr Asn Gln Ile Asp Pro His Arg Val Phe Thr
        545             550             555             560

        Asn Gly Phe Met Asp Lys Leu Leu Pro
                565
```

(2) INFORMATION ZU SEQ ID NO: 24:

    (i) SEQUENZ CHARAKTERISTIKA:

(A) LÄNGE: 1728 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 19..1728

(xi) SEQUENZBESCHREIBUNG: SEQ ID NO: 24:

```
AATTTGGAGG GGAACATT ATG AGT AAT CAT CAC CAT GGG CAT GCC TCG ACC        51
                    Met Ser Asn His His His Gly His Ala Ser Thr
                     1           5                  10

GGG CCG GTC GCG CCG CTT CCG ACG CCG CCG AAC TTC CCG AAC GAC ATC        99
Gly Pro Val Ala Pro Leu Pro Thr Pro Pro Asn Phe Pro Asn Asp Ile
            15              20              25

GCG CTG TTC CAG CAG GCG TAC CAG AAC TGG TCC AAG GAG ATC ATG CTG       147
Ala Leu Phe Gln Gln Ala Tyr Gln Asn Trp Ser Lys Glu Ile Met Leu
            30              35              40

GAC GCC ACT TGG GTC TGC TCG CCC AAG ACG CCG CAG GAT GTC GTT CGC       195
Asp Ala Thr Trp Val Cys Ser Pro Lys Thr Pro Gln Asp Val Val Arg
        45              50              55

CTT GCC AAC TGG GCG CAC GAG CAC GAC TAC AAG ATC CGC CCG CGC GGC       243
Leu Ala Asn Trp Ala His Glu His Asp Tyr Lys Ile Arg Pro Arg Gly
    60              65              7C                  75

GCG ATG CAC GGC TGG ACC CCG CTC ACC GTG GAG AAG GGG GCC AAC GTC       291
Ala Met His Gly Trp Thr Pro Leu Thr Val Glu Lys Gly Ala Asn Val
                80              85              90

GAG AAG GTG ATC CTC GCC GAC ACG ATG ACG CAT CTG AAC GGC ATC ACG       339
Glu Lys Val Ile Leu Ala Asp Thr Met Thr His Leu Asn Gly Ile Thr
            95              100             105

GTG AAC ACG GGC GGC CCC GTG GCT ACC GTC ACC GCC GGT GCC GGC GCC       387
Val Asn Thr Gly Gly Pro Val Ala Thr Val Thr Ala Gly Ala Gly Ala
            110             115             120

AGC ATC GAG GCG ATC GTC ACC GAA CTG CAG AAG CAC GAC CTC GGC TGG       435
Ser Ile Glu Ala Ile Val Thr Glu Leu Gln Lys His Asp Leu Gly Trp
    125             130             135

GCC AAC CTG CCC GCT CCG GGT GTG CTG TCG ATC GGT GGC GCC CTT GCG       483
Ala Asn Leu Pro Ala Pro Gly Val Leu Ser Ile Gly Gly Ala Leu Ala
140             145             150             155

GTC AAC GCG CAC GGT GCG GCG CTG CCG GCC GTC GGC CAG ACC ACG CTG       531
Val Asn Ala His Gly Ala Ala Leu Pro Ala Val Gly Gln Thr Thr Leu
                160             165             170

CCC GGT CAC ACC TAC GGT TCG CTG AGC AAC CTG GTC ACC GAG CTG ACC       579
Pro Gly His Thr Tyr Gly Ser Leu Ser Asn Leu Val Thr Glu Leu Thr
                175             180             185
```

```
GCG GTC GTC TGG AAC GGC ACC ACC TAC GCA CTC GAG ACG TAC CAG CGC          627
Ala Val Val Trp Asn Gly Thr Thr Tyr Ala Leu Glu Thr Tyr Gln Arg
        190             195                 200

AAC GAT CCT CGG ATC ACC CCA CTG CTC ACC AAC CTC GGG CGC TGC TTC          675
Asn Asp Pro Arg Ile Thr Pro Leu Leu Thr Asn Leu Gly Arg Cys Phe
        205             210             215

CTG ACC TCG GTG ACG ATG CAG GCC GGC CCC AAC TTC CGT CAG CGG TGC          723
Leu Thr Ser Val Thr Met Gln Ala Gly Pro Asn Phe Arg Gln Arg Cys
220             225                 230             235

CAG AGC TAC ACC GAC ATC CCG TGG CGG GAA CTG TTC GCG CCG AAG GGC          771
Gln Ser Tyr Thr Asp Ile Pro Trp Arg Glu Leu Phe Ala Pro Lys Gly
            240                 245             250

GCC GAC GGC CGC ACG TTC GAG AAG TTC GTC GCG GAA TCG GGC GGC GCC          819
Ala Asp Gly Arg Thr Phe Glu Lys Phe Val Ala Glu Ser Gly Gly Ala
            255                 260             265

GAG GCG ATC TGG TAC CCG TTC ACC GAG AAG CCG TGG ATG AAG GTG TGG          867
Glu Ala Ile Trp Tyr Pro Phe Thr Glu Lys Pro Trp Met Lys Val Trp
            270                 275             280

ACG GTC TCG CCG ACC AAG CCG GAC TCG TCG AAC GAG GTC GGA AGC CTC          915
Thr Val Ser Pro Thr Lys Pro Asp Ser Ser Asn Glu Val Gly Ser Leu
        285                 290             295

GGC TCG GCG GGC TCC CTC GTC GGC AAG CCT CCG CAG GCG CGT GAG GTC          963
Gly Ser Ala Gly Ser Leu Val Gly Lys Pro Pro Gln Ala Arg Glu Val
300                 305                 310             315

TCC GGC CCG TAC AAC TAC ATC TTC TCC GAC AAC CTG CCG GAG CCC ATC          1011
Ser Gly Pro Tyr Asn Tyr Ile Phe Ser Asp Asn Leu Pro Glu Pro Ile
            320                 325                 330

ACC GAC ATG ATC GGC GCC ATC AAC GCC GGA AAC CCC GGA ATC GCA CCG          1059
Thr Asp Met Ile Gly Ala Ile Asn Ala Gly Asn Pro Gly Ile Ala Pro
            335                 340             345

CTG TTC GGC CCG GCG ATG TAC GAG ATC ACC AAG CTC GGG CTG GCC GCG          1107
Leu Phe Gly Pro Ala Met Tyr Glu Ile Thr Lys Leu Gly Leu Ala Ala
            350                 355             360

ACG AAT GCC AAC GAC ATC TGG GGC TGG TCG AAG GAC GTC CAG TTC TAC          1155
Thr Asn Ala Asn Asp Ile Trp Gly Trp Ser Lys Asp Val Gln Phe Tyr
        365                 370             375

ATC AAG GCC ACG ACG TTG CGA CTC ACC GAG GGC GGC GGC GCC GTC GTC          1203
Ile Lys Ala Thr Thr Leu Arg Leu Thr Glu Gly Gly Gly Ala Val Val
380                 385                 390             395

ACG AGC CGC GCC AAC ATC GCG ACC GTG ATC AAC GAC TTC ACC GAG TGG          1251
Thr Ser Arg Ala Asn Ile Ala Thr Val Ile Asn Asp Phe Thr Glu Trp
            400                 405             410

TTC CAC GAG CGC ATC GAG TTC TAC CGC GCG AAG GGC GAG TTC CCG CTC          1299
Phe His Glu Arg Ile Glu Phe Tyr Arg Ala Lys Gly Glu Phe Pro Leu
            415                 420             425

AAC GGT CCG GTC GAG ATC CGC TGC TGC GGG CTC GAT CAG GCA GCC GAC          1347
Asn Gly Pro Val Glu Ile Arg Cys Cys Gly Leu Asp Gln Ala Ala Asp
        430                 435             440
```

```
GTC AAG GTG CCG TCG GTG GGC CCG CCG ACC ATC TCG GCG ACC CGT CCG     1395
Val Lys Val Pro Ser Val Gly Pro Pro Thr Ile Ser Ala Thr Arg Pro
        445                 450             455

CGT CCG GAT CAT CCG GAC TGG GAC GTC GCG ATC TGG CTG AAC GTT CTC     1443
Arg Pro Asp His Pro Asp Trp Asp Val Ala Ile Trp Leu Asn Val Leu
460             465                 470                 475

GGT GTT CCG GGC ACC CCC GGC ATG TTC GAG TTC TAC CGC GAG ATG GAG     1491
Gly Val Pro Gly Thr Pro Gly Met Phe Glu Phe Tyr Arg Glu Met Glu
                480             485                 490

CAG TGG ATG CGG AGC CAC TAC AAC AAC GAC GAC GCC ACC TTC CGG CCC     1539
Gln Trp Met Arg Ser His Tyr Asn Asn Asp Asp Ala Thr Phe Arg Pro
            495                 500             505

GAG TGG TCG AAG GGG TGG GCG TTC GGT CCC GAC CCG TAC ACC GAC AAC     1587
Glu Trp Ser Lys Gly Trp Ala Phe Gly Pro Asp Pro Tyr Thr Asp Asn
        510                 515             520

GAC ATC GTC ACG AAC AAG ATG CGC GCC ACC TAC ATC GAA GGT GTC CCG     1635
Asp Ile Val Thr Asn Lys Met Arg Ala Thr Tyr Ile Glu Gly Val Pro
        525                 530             535

ACG ACC GAG AAC TGG GAC ACC GCG CGC GCT CGG TAC AAC CAG ATC GAC     1683
Thr Thr Glu Asn Trp Asp Thr Ala Arg Ala Arg Tyr Asn Gln Ile Asp
540             545                 550                 555

CCG CAT CGC GTG TTC ACC AAC GGA TTC ATG GAC AAG CTG CTT CCG         1728
Pro His Arg Val Phe Thr Asn Gly Phe Met Asp Lys Leu Leu Pro
                560             565                 570
```

(2) INFORMATION ZU SEQ ID NO: 25:

    (i) SEQUENZ CHARAKTERISTIKA:

        (A) LANGE: 570 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 25:

```
Met Ser Asn His His His Gly His Ala Ser Thr Gly Pro Val Ala Pro
 1               5               10              15

Leu Pro Thr Pro Pro Asn Phe Pro Asn Asp Ile Ala Leu Phe Gln Gln
        20              25                      30

Ala Tyr Gln Asn Trp Ser Lys Glu Ile Met Leu Asp Ala Thr Trp Val
        35              40                      45

Cys Ser Pro Lys Thr Pro Gln Asp Val Val Arg Leu Ala Asn Trp Ala
    50              55                      60

His Glu His Asp Tyr Lys Ile Arg Pro Arg Gly Ala Met His Gly Trp
65              70                      75                      80

Thr Pro Leu Thr Val Glu Lys Gly Ala Asn Val Glu Lys Val Ile Leu
                85                      90                      95
```

```
Ala Asp Thr Met Thr His Leu Asn Gly Ile Thr Val Asn Thr Gly Gly
            100             105             110

Pro Val Ala Thr Val Thr Ala Gly Ala Gly Ala Ser Ile Glu Ala Ile
        115             120             125

Val Thr Glu Leu Gln Lys His Asp Leu Gly Trp Ala Asn Leu Pro Ala
    130             135             140

Pro Gly Val Leu Ser Ile Gly Gly Ala Leu Ala Val Asn Ala His Gly
145             150             155             160

Ala Ala Leu Pro Ala Val Gly Gln Thr Thr Leu Pro Gly His Thr Tyr
            165             170             175

Gly Ser Leu Ser Asn Leu Val Thr Glu Leu Thr Ala Val Val Trp Asn
            180             185             190

Gly Thr Thr Tyr Ala Leu Glu Thr Tyr Gln Arg Asn Asp Pro Arg Ile
        195             200             205

Thr Pro Leu Leu Thr Asn Leu Gly Arg Cys Phe Leu Thr Ser Val Thr
    210             215             220

Met Gln Ala Gly Pro Asn Phe Arg Gln Arg Cys Gln Ser Tyr Thr Asp
225             230             235             240

Ile Pro Trp Arg Glu Leu Phe Ala Pro Lys Gly Ala Asp Gly Arg Thr
            245             250             255

Phe Glu Lys Phe Val Ala Glu Ser Gly Gly Ala Glu Ala Ile Trp Tyr
        260             265             270

Pro Phe Thr Glu Lys Pro Trp Met Lys Val Trp Thr Val Ser Pro Thr
        275             280             285

Lys Pro Asp Ser Ser Asn Glu Val Gly Ser Leu Gly Ser Ala Gly Ser
    290             295             300

Leu Val Gly Lys Pro Pro Gln Ala Arg Glu Val Ser Gly Pro Tyr Asn
305             310             315             320

Tyr Ile Phe Ser Asp Asn Leu Pro Glu Pro Ile Thr Asp Met Ile Gly
            325             330             335

Ala Ile Asn Ala Gly Asn Pro Gly Ile Ala Pro Leu Phe Gly Pro Ala
        340             345             350

Met Tyr Glu Ile Thr Lys Leu Gly Leu Ala Ala Thr Asn Ala Asn Asp
        355             360             365

Ile Trp Gly Trp Ser Lys Asp Val Gln Phe Tyr Ile Lys Ala Thr Thr
    370             375             380

Leu Arg Leu Thr Glu Gly Gly Gly Ala Val Val Thr Ser Arg Ala Asn
385             390             395             400

Ile Ala Thr Val Ile Asn Asp Phe Thr Glu Trp Phe His Glu Arg Ile
            405             410             415

Glu Phe Tyr Arg Ala Lys Gly Glu Phe Pro Leu Asn Gly Pro Val Glu
            420             425             430
```

39

```
Ile Arg Cys Cys Gly Leu Asp Gln Ala Ala Asp Val Lys Val Pro Ser
        435             440             445

Val Gly Pro Pro Thr Ile Ser Ala Thr Arg Pro Arg Pro Asp His Pro
    450             455             460

Asp Trp Asp Val Ala Ile Trp Leu Asn Val Leu Gly Val Pro Gly Thr
465             470             475             480

Pro Gly Met Phe Glu Phe Tyr Arg Glu Met Glu Gln Trp Met Arg Ser
            485             490             495

His Tyr Asn Asn Asp Asp Ala Thr Phe Arg Pro Glu Trp Ser Lys Gly
            500             505             510

Trp Ala Phe Gly Pro Asp Pro Tyr Thr Asp Asn Asp Ile Val Thr Asn
        515             520             525

Lys Met Arg Ala Thr Tyr Ile Glu Gly Val Pro Thr Thr Glu Asn Trp
    530             535             540

Asp Thr Ala Arg Ala Arg Tyr Asn Gln Ile Asp Pro His Arg Val Phe
545             550             555             560

Thr Asn Gly Phe Met Asp Lys Leu Leu Pro
            565             570
```

(2) INFORMATION ZU SEQ ID NO: 26:

(i) SEQUENZ CHARAKTERISTIKA:

(A) LÄNGE: 1741 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 20..1741

(xi) SEQUENZBESCHREIBUNG: SEQ ID NO: 26:

```
GAATTTAAGG GGAACATCG ATG AGT AAT ACG CGT AAA CGC AAG CGC CGT ACG        52
                     Met Ser Asn Thr Arg Lys Arg Lys Arg Arg Thr
                      1               5                   10


CAT GCC TCG ACC GGG CCG GTC GCG CCG CTT CCG ACG CCG CCG AAC TTC        100
His Ala Ser Thr Gly Pro Val Ala Pro Leu Pro Thr Pro Pro Asn Phe
             15              20              25


CCG AAC GAC ATC GCG CTG TTC CAG CAG GCG TAC CAG AAC TGG TCC AAG        148
Pro Asn Asp Ile Ala Leu Phe Gln Gln Ala Tyr Gln Asn Trp Ser Lys
             30              35              40


GAG ATC ATG CTG GAC GCC ACT TGG GTC TGC TCG CCC AAG ACG CCG CAG        196
Glu Ile Met Leu Asp Ala Thr Trp Val Cys Ser Pro Lys Thr Pro Gln
     45              50              55


GAT GTC GTT CGC CTT GCC AAC TGG GCG CAC GAG CAC GAC TAC AAG ATC        244
Asp Val Val Arg Leu Ala Asn Trp Ala His Glu His Asp Tyr Lys Ile
 60              65              70              75
```

```
CGC CCG CGC GGC GCG ATG CAC GGC TGG ACC CCG CTC ACC GTG GAG AAG        292
Arg Pro Arg Gly Ala Met His Gly Trp Thr Pro Leu Thr Val Glu Lys
            80              85                      90

GGG GCC AAC GTC GAG AAG GTG ATC CTC GCC GAC ACG ATG ACG CAT CTG        340
Gly Ala Asn Val Glu Lys Val Ile Leu Ala Asp Thr Met Thr His Leu
            95              100                     105

AAC GGC ATC ACG GTG AAC ACG GGC GGC CCC GTG GCT ACC GTC ACC GCC        388
Asn Gly Ile Thr Val Asn Thr Gly Gly Pro Val Ala Thr Val Thr Ala
            110             115                     120

GGT GCC GGC GCC AGC ATC GAG GCG ATC GTC ACC GAA CTG CAG AAG CAC        436
Gly Ala Gly Ala Ser Ile Glu Ala Ile Val Thr Glu Leu Gln Lys His
    125             130                     135

GAC CTC GGC TGG GCC AAC CTG CCC GCT CCG GGT GTG CTG TCG ATC GGT        484
Asp Leu Gly Trp Ala Asn Leu Pro Ala Pro Gly Val Leu Ser Ile Gly
140             145                     150                     155

GGC GCC CTT GCG GTC AAC GCG CAC GGT GCG GCG CTG CCG GCC GTC GGC        532
Gly Ala Leu Ala Val Asn Ala His Gly Ala Ala Leu Pro Ala Val Gly
            160             165                     170

CAG ACC ACG CTG CCC GGT CAC ACC TAC GGT TCG CTG AGC AAC CTG GTC        580
Gln Thr Thr Leu Pro Gly His Thr Tyr Gly Ser Leu Ser Asn Leu Val
            175             180                     185

ACC GAG CTG ACC GCG GTC GTC TGG AAC GGC ACC ACC TAC GCA CTC GAG        628
Thr Glu Leu Thr Ala Val Val Trp Asn Gly Thr Thr Tyr Ala Leu Glu
            190             195                     200

ACG TAC CAG CGC AAC GAT CCT CGG ATC ACC CCA CTG CTC ACC AAC CTC        676
Thr Tyr Gln Arg Asn Asp Pro Arg Ile Thr Pro Leu Leu Thr Asn Leu
            205             210                     215

GGG CGC TGC TTC CTG ACC TCG GTG ACG ATG CAG GCC GGC CCC AAC TTC        724
Gly Arg Cys Phe Leu Thr Ser Val Thr Met Gln Ala Gly Pro Asn Phe
220             225                     230                     235

CGT CAG CGG TGC CAG AGC TAC ACC GAC ATC CCG TGG CGG GAA CTG TTC        772
Arg Gln Arg Cys Gln Ser Tyr Thr Asp Ile Pro Trp Arg Glu Leu Phe
            240                     245                     250

GCG CCG AAG GGC GCC GAC GGC CGC ACG TTC GAG AAG TTC GTC GCG GAA        820
Ala Pro Lys Gly Ala Asp Gly Arg Thr Phe Glu Lys Phe Val Ala Glu
            255                     260                     265

TCG GGC GGC GCC GAG GCG ATC TGG TAC CCG TTC ACC GAG AAG CCG TGG        868
Ser Gly Gly Ala Glu Ala Ile Trp Tyr Pro Phe Thr Glu Lys Pro Trp
            270                     275                     280

ATG AAG GTG TGG ACG GTC TCG CCG ACC AAG CCG GAC TCG TCG AAC GAG        916
Met Lys Val Trp Thr Val Ser Pro Thr Lys Pro Asp Ser Ser Asn Glu
            285                     290                     295

GTC GGA AGC CTC GGC TCG GCG GGC TCC CTC GTC GGC AAG CCT CCG CAG        964
Val Gly Ser Leu Gly Ser Ala Gly Ser Leu Val Gly Lys Pro Pro Gln
300             305                     310                     315

GCG CGT GAG GTC TCC GGC CCG TAC AAC TAC ATC TTC TCC GAC AAC CTG        1012
Ala Arg Glu Val Ser Gly Pro Tyr Asn Tyr Ile Phe Ser Asp Asn Leu
            320                     325                     330
```

```
CCG GAG CCC ATC ACC GAC ATG ATC GGC GCC ATC AAC GCC GGA AAC CCC        1060
Pro Glu Pro Ile Thr Asp Met Ile Gly Ala Ile Asn Ala Gly Asn Pro
            335             340             345

GGA ATC GCA CCG CTG TTC GGC CCG GCG ATG TAC GAG ATC ACC AAG CTC        1108
Gly Ile Ala Pro Leu Phe Gly Pro Ala Met Tyr Glu Ile Thr Lys Leu
            350             355             360

GGG CTG GCC GCG ACG AAT GCC AAC GAC ATC TGG GGC TGG TCG AAG GAC        1156
Gly Leu Ala Ala Thr Asn Ala Asn Asp Ile Trp Gly Trp Ser Lys Asp
            365             370             375

GTC CAG TTC TAC ATC AAG GCC ACG ACG TTG CGA CTC ACC GAG GGC GGC        1204
Val Gln Phe Tyr Ile Lys Ala Thr Thr Leu Arg Leu Thr Glu Gly Gly
380             385             390             395

GGC GCC GTC GTC ACG AGC CGC GCC AAC ATC GCG ACC GTG ATC AAC GAC        1252
Gly Ala Val Val Thr Ser Arg Ala Asn Ile Ala Thr Val Ile Asn Asp
            400             405             410

TTC ACC GAG TGG TTC CAC GAG CGC ATC GAG TTC TAC CGC GCG AAG GGC        1300
Phe Thr Glu Trp Phe His Glu Arg Ile Glu Phe Tyr Arg Ala Lys Gly
            415             420             425

GAG TTC CCG CTC AAC GGT CCG GTC GAG ATC CGC TGC TGC GGG CTC GAT        1348
Glu Phe Pro Leu Asn Gly Pro Val Glu Ile Arg Cys Cys Gly Leu Asp
            430             435             440

CAG GCA GCC GAC GTC AAG GTG CCG TCG GTG GGC CCG CCG ACC ATC TCG        1396
Gln Ala Ala Asp Val Lys Val Pro Ser Val Gly Pro Pro Thr Ile Ser
            445             450             455

GCG ACC CGT CCG CGT CCG GAT CAT CCG GAC TGG GAC GTC GCG ATC TGG        1444
Ala Thr Arg Pro Arg Pro Asp His Pro Asp Trp Asp Val Ala Ile Trp
460             465             470             475

CTG AAC GTT CTC GGT GTT CCG GGC ACC CCC GGC ATG TTC GAG TTC TAC        1492
Leu Asn Val Leu Gly Val Pro Gly Thr Pro Gly Met Phe Glu Phe Tyr
            480             485             490

CGC GAG ATG GAG CAG TGG ATG CGG AGC CAC TAC AAC AAC GAC GAC GCC        1540
Arg Glu Met Glu Gln Trp Met Arg Ser His Tyr Asn Asn Asp Asp Ala
            495             500             505

ACC TTC CGG CCC GAG TGG TCG AAG GGG TGG GCG TTC GGT CCC GAC CCG        1588
Thr Phe Arg Pro Glu Trp Ser Lys Gly Trp Ala Phe Gly Pro Asp Pro
            510             515             520

TAC ACC GAC AAC GAC ATC GTC ACG AAC AAG ATG CGC GCC ACC TAC ATC        1636
Tyr Thr Asp Asn Asp Ile Val Thr Asn Lys Met Arg Ala Thr Tyr Ile
            525             530             535

GAA GGT GTC CCG ACG ACC GAG AAC TGG GAC ACC GCG CGC GCT CGG TAC        1684
Glu Gly Val Pro Thr Thr Glu Asn Trp Asp Thr Ala Arg Ala Arg Tyr
540             545             550             555

AAC CAG ATC GAC CCG CAT CGC GTG TTC ACC AAC GGA TTC ATG GAC AAG        1732
Asn Gln Ile Asp Pro His Arg Val Phe Thr Asn Gly Phe Met Asp Lys
            560             565             570

CTG CTT CCG                                                             1741
Leu Leu Pro
```

(2) INFORMATION ZU SEQ ID NO: 27:

   (i) SEQUENZ CHARAKTERISTIKA:

      (A) LANGE: 574 Aminosäuren
      (B) ART: Aminosäure
      (D) TOPOLOGIE: linear

   (ii) ART DES MOLEKÜLS: Protein
   (xi) SEQUENZBESCHREIBUNG: SEQ ID NO: 27:

```
Met  Ser  Asn  Thr  Arg  Lys  Arg  Lys  Arg  Arg  Thr  His  Ala  Ser  Thr  Gly
 1               5                    10                       15

Pro  Val  Ala  Pro  Leu  Pro  Thr  Pro  Pro  Asn  Phe  Pro  Asn  Asp  Ile  Ala
          20                    25                            30

Leu  Phe  Gln  Gln  Ala  Tyr  Gln  Asn  Trp  Ser  Lys  Glu  Ile  Met  Leu  Asp
          35                    40                       45

Ala  Thr  Trp  Val  Cys  Ser  Pro  Lys  Thr  Pro  Gln  Asp  Val  Val  Arg  Leu
     50                    55                       60

Ala  Asn  Trp  Ala  His  Glu  His  Asp  Tyr  Lys  Ile  Arg  Pro  Arg  Gly  Ala .
 65                    70                    75                            80

Met  His  Gly  Trp  Thr  Pro  Leu  Thr  Val  Glu  Lys  Gly  Ala  Asn  Val  Glu
               85                    90                            95

Lys  Val  Ile  Leu  Ala  Asp  Thr  Met  Thr  His  Leu  Asn  Gly  Ile  Thr  Val
               100                  105                       110

Asn  Thr  Gly  Gly  Pro  Val  Ala  Thr  Val  Thr  Ala  Gly  Ala  Gly  Ala  Ser
          115                  120                       125

Ile  Glu  Ala  Ile  Val  Thr  Glu  Leu  Gln  Lys  His  Asp  Leu  Gly  Trp  Ala
     130                  135                       140

Asn  Leu  Pro  Ala  Pro  Gly  Val  Leu  Ser  Ile  Gly  Gly  Ala  Leu  Ala  Val
145                  150                  155                       160

Asn  Ala  His  Gly  Ala  Ala  Leu  Pro  Ala  Val  Gly  Gln  Thr  Thr  Leu  Pro
               165                  170                       175

Gly  His  Thr  Tyr  Gly  Ser  Leu  Ser  Asn  Leu  Val  Thr  Glu  Leu  Thr  Ala
               180                  185                       190

Val  Val  Trp  Asn  Gly  Thr  Thr  Tyr  Ala  Leu  Glu  Thr  Tyr  Gln  Arg  Asn
          195                  200                       205

Asp  Pro  Arg  Ile  Thr  Pro  Leu  Leu  Thr  Asn  Leu  Gly  Arg  Cys  Phe  Leu
     210                  215                       220

Thr  Ser  Val  Thr  Met  Gln  Ala  Gly  Pro  Asn  Phe  Arg  Gln  Arg  Cys  Gln
225                  230                  235                       240

Ser  Tyr  Thr  Asp  Ile  Pro  Trp  Arg  Glu  Leu  Phe  Ala  Pro  Lys  Gly  Ala
               245                  250                       255

Asp  Gly  Arg  Thr  Phe  Glu  Lys  Phe  Val  Ala  Glu  Ser  Gly  Gly  Ala  Glu
               260                  265                       270
```

```
Ala Ile Trp Tyr Pro Phe Thr Glu Lys Pro Trp Met Lys Val Trp Thr
    275                 280                 285

Val Ser Pro Thr Lys Pro Asp Ser Ser Asn Glu Val Gly Ser Leu Gly
    290             295                 300

Ser Ala Gly Ser Leu Val Gly Lys Pro Pro Gln Ala Arg Glu Val Ser
305                 310                 315                 320

Gly Pro Tyr Asn Tyr Ile Phe Ser Asp Asn Leu Pro Glu Pro Ile Thr
            325                 330                 335

Asp Met Ile Gly Ala Ile Asn Ala Gly Asn Pro Gly Ile Ala Pro Leu
        340                 345                 350

Phe Gly Pro Ala Met Tyr Glu Ile Thr Lys Leu Gly Leu Ala Ala Thr
    355                 360                 365

Asn Ala Asn Asp Ile Trp Gly Trp Ser Lys Asp Val Gln Phe Tyr Ile
370                 375                 380

Lys Ala Thr Thr Leu Arg Leu Thr Glu Gly Gly Gly Ala Val Val Thr
385                 390                 395                 400

Ser Arg Ala Asn Ile Ala Thr Val Ile Asn Asp Phe Thr Glu Trp Phe
            405                 410                 415

His Glu Arg Ile Glu Phe Tyr Arg Ala Lys Gly Glu Phe Pro Leu Asn
        420                 425                 430

Gly Pro Val Glu Ile Arg Cys Cys Gly Leu Asp Gln Ala Ala Asp Val
        435                 440                 445

Lys Val Pro Ser Val Gly Pro Pro Thr Ile Ser Ala Thr Arg Pro Arg
    450                 455                 460

Pro Asp His Pro Asp Trp Asp Val Ala Ile Trp Leu Asn Val Leu Gly
465                 470                 475                 480

Val Pro Gly Thr Pro Gly Met Phe Glu Phe Tyr Arg Glu Met Glu Gln
            485                 490                 495

Trp Met Arg Ser His Tyr Asn Asn Asp Asp Ala Thr Phe Arg Pro Glu
        500                 505                 510

Trp Ser Lys Gly Trp Ala Phe Gly Pro Asp Pro Tyr Thr Asp Asn Asp
        515                 520                 525

Ile Val Thr Asn Lys Met Arg Ala Thr Tyr Ile Glu Gly Val Pro Thr
    530                 535                 540

Thr Glu Asn Trp Asp Thr Ala Arg Ala Arg Tyr Asn Gln Ile Asp Pro
545                 550                 555                 560

His Arg Val Phe Thr Asn Gly Phe Met Asp Lys Leu Leu Pro
            565                 570
```

(2) INFORMATION ZU SEQ ID NO: 28:

(i) SEQUENZ CHARAKTERISTIKA:

(A) LÄNGE: 1731 Basenpaare
(B) ART: Nukleinsäure
(C) STRANGFORM: Einzel
(D) TOPOLOGIE: linear

(ix) MERKMALE:

(A) NAME/SCHLÜSSEL: CDS
(B) LAGE: 25..1731

(xi) SEQUENZBESCHREIBUNG: SEQ ID NO: 28:

```
GAATTCACAC AGGAAACAGA ATTC ATG GTT ATG CAC CAT GGG CAT GCC TCG        51
                           Met Val Met His His Gly His Ala Ser
                            1                   5

ACC GGG CCG GTC GCG CCG CTT CCG ACG CCG CCG AAC TTC CCG AAC GAC       99
Thr Gly Pro Val Ala Pro Leu Pro Thr Pro Pro Asn Phe Pro Asn Asp
 10              15                  20                  25

ATC GCG CTG TTC CAG CAG GCG TAC CAG AAC TGG TCC AAG GAG ATC ATG      147
Ile Ala Leu Phe Gln Gln Ala Tyr Gln Asn Trp Ser Lys Glu Ile Met
             30                  35              40

CTG GAC GCC ACT TGG GTC TGC TCG CCC AAG ACG CCG CAG GAT GTC GTT      195
Leu Asp Ala Thr Trp Val Cys Ser Pro Lys Thr Pro Gln Asp Val Val
             45                  50                  55

CGC CTT GCC AAC TGG GCG CAC GAG CAC GAC TAC AAG ATC CGC CCG CGC      243
Arg Leu Ala Asn Trp Ala His Glu His Asp Tyr Lys Ile Arg Pro Arg
         60                  65                  70

GGC GCG ATG CAC GGC TGG ACC CCG CTC ACC GTG GAG AAG GGG GCC AAC      291
Gly Ala Met His Gly Trp Thr Pro Leu Thr Val Glu Lys Gly Ala Asn
     75                  80                  85

GTC GAG AAG GTG ATC CTC GCC GAC ACG ATG ACG CAT CTG AAC GGC ATC      339
Val Glu Lys Val Ile Leu Ala Asp Thr Met Thr His Leu Asn Gly Ile
 90                  95                  100                 105

ACG GTG AAC ACG GGC GGC CCC GTG GCT ACC GTC ACC GCC GGT GCC GGC      387
Thr Val Asn Thr Gly Gly Pro Val Ala Thr Val Thr Ala Gly Ala Gly
                 110                 115                 120

GCC AGC ATC GAG GCG ATC GTC ACC GAA CTG CAG AAG CAC GAC CTC GGC      435
Ala Ser Ile Glu Ala Ile Val Thr Glu Leu Gln Lys His Asp Leu Gly
             125                 130                 135

TGG GCC AAC CTG CCC GCT CCG GGT GTG CTG TCG ATC GGT GGC GCC CTT      483
Trp Ala Asn Leu Pro Ala Pro Gly Val Leu Ser Ile Gly Gly Ala Leu
             140                 145                 150

GCG GTC AAC GCG CAC GGT GCG GCG CTG CCG GCC GTC GGC CAG ACC ACG      531
Ala Val Asn Ala His Gly Ala Ala Leu Pro Ala Val Gly Gln Thr Thr
     155                 160                 165

CTG CCC GGT CAC ACC TAC GGT TCG CTG AGC AAC CTG GTC ACC GAG CTG      579
Leu Pro Gly His Thr Tyr Gly Ser Leu Ser Asn Leu Val Thr Glu Leu
170                 175                 180                 185
```

```
ACC GCG GTC GTC TGG AAC GGC ACC ACC TAC GCA CTC GAG ACG TAC CAG        627
Thr Ala Val Val Trp Asn Gly Thr Thr Tyr Ala Leu Glu Thr Tyr Gln
            190                 195                 200

CGC AAC GAT CCT CGG ATC ACC CCA CTG CTC ACC AAC CTC GGG CGC TGC        675
Arg Asn Asp Pro Arg Ile Thr Pro Leu Leu Thr Asn Leu Gly Arg Cys
            205                 210                 215

TTC CTG ACC TCG GTG ACG ATG CAG GCC GGC CCC AAC TTC CGT CAG CGG        723
Phe Leu Thr Ser Val Thr Met Gln Ala Gly Pro Asn Phe Arg Gln Arg
            220                 225                 230

TGC CAG AGC TAC ACC GAC ATC CCG TGG CGG GAA CTG TTC GCG CCG AAG        771
Cys Gln Ser Tyr Thr Asp Ile Pro Trp Arg Glu Leu Phe Ala Pro Lys
            235                 240                 245

GGC GCC GAC GGC CGC ACG TTC GAG AAG TTC GTC GCG GAA TCG GGC GGC        819
Gly Ala Asp Gly Arg Thr Phe Glu Lys Phe Val Ala Glu Ser Gly Gly
250                 255                 260                 265

GCC GAG GCG ATC TGG TAC CCG TTC ACC GAG AAG CCG TGG ATG AAG GTG        867
Ala Glu Ala Ile Trp Tyr Pro Phe Thr Glu Lys Pro Trp Met Lys Val
            270                 275             -       280

TGG ACG GTC TCG CCG ACC AAG CCG GAC TCG TCG AAC GAG GTC GGA AGC        915
Trp Thr Val Ser Pro Thr Lys Pro Asp Ser Ser Asn Glu Val Gly Ser
            285                 290                 295

CTC GGC TCG GCG GGC TCC CTC GTC GGC AAG CCT CCG CAG GCG CGT GAG        963
Leu Gly Ser Ala Gly Ser Leu Val Gly Lys Pro Pro Gln Ala Arg Glu
            300                 305                 310

GTC TCC GGC CCG TAC AAC TAC ATC TTC TCC GAC AAC CTG CCG GAG CCC        1011
Val Ser Gly Pro Tyr Asn Tyr Ile Phe Ser Asp Asn Leu Pro Glu Pro
            315                 320                 325

ATC ACC GAC ATG ATC GGC GCC ATC AAC GCC GGA AAC CCC GGA ATC GCA        1059
Ile Thr Asp Met Ile Gly Ala Ile Asn Ala Gly Asn Pro Gly Ile Ala
330                 335                 340                 345

CCG CTG TTC GGC CCG GCG ATG TAC GAG ATC ACC AAG CTC GGG CTG GCC        1107
Pro Leu Phe Gly Pro Ala Met Tyr Glu Ile Thr Lys Leu Gly Leu Ala
            350                 355                 360

GCG ACG AAT GCC AAC GAC ATC TGG GGC TGG TCG AAG GAC GTC CAG TTC        1155
Ala Thr Asn Ala Asn Asp Ile Trp Gly Trp Ser Lys Asp Val Gln Phe
            365                 370                 375

TAC ATC AAG GCC ACG ACG TTG CGA CTC ACC GAG GGC GGC GGC GCC GTC        1203
Tyr Ile Lys Ala Thr Thr Leu Arg Leu Thr Glu Gly Gly Gly Ala Val
            380                 385                 390

GTC ACG AGC CGC GCC AAC ATC GCG ACC GTG ATC AAC GAC TTC ACC GAG        1251
Val Thr Ser Arg Ala Asn Ile Ala Thr Val Ile Asn Asp Phe Thr Glu
            395                 400                 405

TGG TTC CAC GAG CGC ATC GAG TTC TAC CGC GCG AAG GGC GAG TTC CCG        1299
Trp Phe His Glu Arg Ile Glu Phe Tyr Arg Ala Lys Gly Glu Phe Pro
410                 415                 420                 425

CTC AAC GGT CCG GTC GAG ATC CGC TGC TGC GGG CTC GAT CAG GCA GCC        1347
Leu Asn Gly Pro Val Glu Ile Arg Cys Cys Gly Leu Asp Gln Ala Ala
            430                 435                 440
```

48

```
GAC GTC AAG GTG CCG TCG GTG GGC CCG CCG ACC ATC TCG GCG ACC CGT        1395
Asp Val Lys Val Pro Ser Val Gly Pro Pro Thr Ile Ser Ala Thr Arg
            445             450                 455

CCG CGT CCG GAT CAT CCG GAC TGG GAC GTC GCG ATC TGG CTG AAC GTT        1443
Pro Arg Pro Asp His Pro Asp Trp Asp Val Ala Ile Trp Leu Asn Val
            460             465                 470

CTC GGT GTT CCG GGC ACC CCC GGC ATG TTC GAG TTC TAC CGC GAG ATG        1491
Leu Gly Val Pro Gly Thr Pro Gly Met Phe Glu Phe Tyr Arg Glu Met
            475             480                 485

GAG CAG TGG ATG CGG AGC CAC TAC AAC AAC GAC GAC GCC ACC TTC CGG        1539
Glu Gln Trp Met Arg Ser His Tyr Asn Asn Asp Asp Ala Thr Phe Arg
490             495                 500                 505

CCC GAG TGG TCG AAG GGG TGG GCG TTC GGT CCC GAC CCG TAC ACC GAC        1587
Pro Glu Trp Ser Lys Gly Trp Ala Phe Gly Pro Asp Pro Tyr Thr Asp
            510             515                 520

AAC GAC ATC GTC ACG AAC AAG ATG CGC GCC ACC TAC ATC GAA GGT GTC        1635
Asn Asp Ile Val Thr Asn Lys Met Arg Ala Thr Tyr Ile Glu Gly Val
            525             530                 535

CCG ACG ACC GAG AAC TGG GAC ACC GCG CGC GCT CGG TAC AAC CAG ATC        1683
Pro Thr Thr Glu Asn Trp Asp Thr Ala Arg Ala Arg Tyr Asn Gln Ile
            540             545                 550

GAC CCG CAT CGC GTG TTC ACC AAC GGA TTC ATG GAC AAG CTG CTT CCG        1731
Asp Pro His Arg Val Phe Thr Asn Gly Phe Met Asp Lys Leu Leu Pro
            555             560                 565
```

(2) INFORMATION ZU SEQ ID NO: 29:

    (i) SEQUENZ CHARAKTERISTIKA:

        (A) LANGE: 569 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 29:

```
Met Val Met His His Gly His Ala Ser Thr Gly Pro Val Ala Pro Leu
 1               5                  10                  15

Pro Thr Pro Pro Asn Phe Pro Asn Asp Ile Ala Leu Phe Gln Gln Ala
         20                  25                  30

Tyr Gln Asn Trp Ser Lys Glu Ile Met Leu Asp Ala Thr Trp Val Cys
         35                  40                  45

Ser Pro Lys Thr Pro Gln Asp Val Val Arg Leu Ala Asn Trp Ala His
     50                  55                  60

Glu His Asp Tyr Lys Ile Arg Pro Arg Gly Ala Met His Gly Trp Thr
 65                  70                  75                  80

Pro Leu Thr Val Glu Lys Gly Ala Asn Val Glu Lys Val Ile Leu Ala
                 85                  90                  95
```

```
Asp Thr Met Thr His Leu Asn Gly Ile Thr Val Asn Thr Gly Gly Pro
            100                 105             110

Val Ala Thr Val Thr Ala Gly Ala Gly Ala Ser Ile Glu Ala Ile Val
        115             120             125

Thr Glu Leu Gln Lys His Asp Leu Gly Trp Ala Asn Leu Pro Ala Pro
    130             135             140

Gly Val Leu Ser Ile Gly Gly Ala Leu Ala Val Asn Ala His Gly Ala
145             150             155             160

Ala Leu Pro Ala Val Gly Gln Thr Thr Leu Pro Gly His Thr Tyr Gly
            165             170             .   175

Ser Leu Ser Asn Leu Val Thr Glu Leu Thr Ala Val Val Trp Asn Gly
        180     .           185             190

Thr Thr Tyr Ala Leu Glu Thr Tyr Gln Arg Asn Asp Pro Arg Ile Thr
    195             200             205

Pro Leu Leu Thr Asn Leu Gly Arg Cys Phe Leu Thr Ser Val Thr Met
    210                 215             220

Gln Ala Gly Pro Asn Phe Arg Gln Arg Cys Gln Ser Tyr Thr Asp Ile
225             230             235             240

Pro Trp Arg Glu Leu Phe Ala Pro Lys Gly Ala Asp Gly Arg Thr Phe
            245             250             255

Glu Lys Phe Val Ala Glu Ser Gly Gly Ala Glu Ala Ile Trp Tyr Pro
            260             265             270

Phe Thr Glu Lys Pro Trp Met Lys Val Trp Thr Val Ser Pro Thr Lys
    275             280             285

Pro Asp Ser Ser Asn Glu Val Gly Ser Leu Gly Ser Ala Gly Ser Leu
    290             295             300

Val Gly Lys Pro Pro Gln Ala Arg Glu Val Ser Gly Pro Tyr Asn Tyr
305             310             315             320

Ile Phe Ser Asp Asn Leu Pro Glu Pro Ile Thr Asp Met Ile Gly Ala
            325             330             335

Ile Asn Ala Gly Asn Pro Gly Ile Ala Pro Leu Phe Gly Pro Ala Met
            340             345             350

Tyr Glu Ile Thr Lys Leu Gly Leu Ala Ala Thr Asn Ala Asn Asp Ile
        355             360             365

Trp Gly Trp Ser Lys Asp Val Gln Phe Tyr Ile Lys Ala Thr Thr Leu
    370             375             380

Arg Leu Thr Glu Gly Gly Gly Ala Val Val Thr Ser Arg Ala Asn Ile
385             390             395             400

Ala Thr Val Ile Asn Asp Phe Thr Glu Trp Phe His Glu Arg Ile Glu
            405             410             415

Phe Tyr Arg Ala Lys Gly Glu Phe Pro Leu Asn Gly Pro Val Glu Ile
        420             425             430
```

51

```
Arg Cys Cys Gly Leu Asp Gln Ala Ala Asp Val Lys Val Pro Ser Val
        435             440             445

Gly Pro Pro Thr Ile Ser Ala Thr Arg Pro Arg Pro Asp His Pro Asp
    450             455             460

Trp Asp Val Ala Ile Trp Leu Asn Val Leu Gly Val Pro Gly Thr Pro
465             470             475             480

Gly Met Phe Glu Phe Tyr Arg Glu Met Glu Gln Trp Met Arg Ser His
            485             490                 495

Tyr Asn Asn Asp Asp Ala Thr Phe Arg Pro Glu Trp Ser Lys Gly Trp
            500             505             510

Ala Phe Gly Pro Asp Pro Tyr Thr Asp Asn Asp Ile Val Thr Asn Lys
        515             520             525

Met Arg Ala Thr Tyr Ile Glu Gly Val Pro Thr Thr Glu Asn Trp Asp
    530             535             540

Thr Ala Arg Ala Arg Tyr Asn Gln Ile Asp Pro His Arg Val Phe Thr
545             550             555             560

Asn Gly Phe Met Asp Lys Leu Leu Pro
            565
```

(2) INFORMATION ZU SEQ ID NO: 30:

    (i) SEQUENZ CHARAKTERISTIKA:

        (A) LÄNGE: 36 Basenpaare
        (B) ART: Nukleinsäure
        (C) STRANGFORM: Einzel
        (D) TOPOLOGIE: linear

    (xi) SEQUENZBESCHREIBUNG: SEQ ID NO: 30:

```
TCGCATGCCT CGACGGGCCC GGTGGCGCCG CTTCCG                    36
```

(2) INFORMATION ZU SEQ ID NO: 31:

    (i) SEQUENZ CHARAKTERISTIKA:

        (A) LÄNGE: 25 Basenpaare
        (B) ART: Nukleinsäure
        (C) STRANGFORM: Einzel
        (D) TOPOLOGIE: linear

    (xi) SEQUENZBESCHREIBUNG: SEQ ID NO: 31:

```
CGTGCTTCTG CAGTTCGGTG ACGAT                                25
```

(2) INFORMATION ZU SEQ ID NO: 32:

(i) SEQUENZ CHARAKTERISTIKA:

(A) LANGE: 39 Basenpaare
(B) ART: Nukleinsäure-
(C) STRANGFORM: Einzel
(D) TOPOLOGIE: linear

(xi) SEQUENZBESCHREIBUNG: SEQ ID NO: 32:

TCCCATGGCA CACAGGAAAC ATCGATGACC ATGATTACG                    39

(2) INFORMATION ZU SEQ ID NO: 33:

(i) SEQUENZ CHARAKTERISTIKA:

(A) LÄNGE: 25 Basenpaare
(B) ART: Nukleinsäure
(C) STRANGFORM: Einzel
(D) TOPOLOGIE: linear

(xi) SEQUENZBESCHREIBUNG: SEQ ID NO: 33:

CGTGCTTCTG CAGTTCGGTG ACGAT                                   25

(2) INFORMATION ZU SEQ ID NO: 34:

(i) SEQUENZ CHARAKTERISTIKA:

(A) LÄNGE: 18 Basenpaare
(B) ART: Nukleinsäure
(C) STRANGFORM: Einzel
(D) TOPOLOGIE: linear

(xi) SEQUENZBESCHREIBUNG: SEQ ID NO: 34:

CGATGCACCA TGGGCATG                                           18